# EUROPEAN PATENT APPLICATION

(11) **EP 4 434 559 A1**
(43) Date of publication of application: **25.09.2024**
(21) Application number: 22893360.2
(22) Date of filing: 01.11.2022
(51) Int. Cl.: A61M 5/20, A61M 5/31, A61M 5/315

(54) **DRUG DELIVERY DEVICE**

(30) Priority: 15.11.2021 RU 2021133254
(71) Applicant: Limited Liability Company "Next Bio", g. Pushkin Saint-Petersburg, 196608 (RU)
(72) Inventor: RODIONOV, Petr Petrovich, Yukki, 188652 (RU); TARASENKO, Fedor Dmitrievich, Saint-Petersburg, 197022 (RU); ZHMAYLO, Michail Alexandrovich, Saint- Petersburg, 194358 (RU)
(74) Representative: ABG Intellectual Property Law, S.L.
(86) International application number: PCT/RU2022/050349
(87) International publication number: WO 2023/085977

(57) **Abstract**

Proposed is a drug delivery device comprising: an outer housing; a drug reservoir; a trigger mechanism; a dose setting mechanism; an inner housing mounted inside the outer housing and functionally connected to the dose setting mechanism; a plunger for expelling a drug from the reservoir; an actuator member which, inside the outer housing, is brought into toothed engagement with the inner housing and into threaded engagement with the plunger in order to move the plunger in accordance with the set drug dose, the actuator member being provided with radial teeth; and a cylindrical member which is provided with a resilient element with a pawl and is fastened inside the outer housing so that the pawl is able to jump over at least one of the radial teeth of the actuator member upon rotation of the actuator member.

## Description

### FIELD OF INVENTION

The present invention generally relates to a medical equipment used for administering or injecting drugs, in particular to drug delivery devices.

### BACKGROUND OF THE INVENTION

Drug delivery devices are widely used in cases where a user needs to self-administer or self-inject a drug, in particular, to inject the drug regularly in set doses in accordance with an individual drug regimen.

Nowadays, drug delivery devices are widely popular among insulin-dependent patients (diabetics) since they allow such patients to manage the disease effectively on their own by performing timely injection of a drug having a required dose into a target injection site on a patient body in accordance with assignments of a consulting health care physician. Such drug delivery devices are easy to use, so that users are not required to have any special medical skills and/or any special medical education in order to set a drug dose to be injected and, then, to inject the set drug dose into the target injection site on the patient body.

The closest prior art (prototype) of the present invention is a drug delivery device (RU 2696459; A61M 5/00; 01.08.2019). It is to note that the drug delivery device disclosed in RU 2696459 comprises: a housing; a cylindrical driving mechanism; a ratchet mechanism comprising a toothed wheel provided with asymmetrical teeth having an inclined and stop surface and mounted on the cylindrical driving mechanism; a pawl; a spring bising the pawl to the toothed wheel; and a cylindrical bearing element, wherein the pawl has three teeth and mounted so as to be radially movable in the cylindrical bearing element installed inside the housing and fixedly connected to the housing, and wherein the toothed wheel is integral with the cylindrical driving mechanism, and wherein the assemblage formed by inserting the pawl and the spring into the cylindrical bearing element and mounted on the driving mechanism is secured together with the driving mechanism in the housing on spacers. When the driving mechanism is rotated in a straightforward direction, the teeth of the toothed wheel and the pawl teeth slides on each other, and the pawl slopes upward and slopes downward again under the action of the spring, thereby providing the reliable engagement and preventing the reverse rotation of the driving mechanism and, therefore, the reverse rotation of the rod and providing with audio feedback when performing the straightforward rotation.

One of disadvantages of the drug delivery device disclosed in RU 2696459 is as follows. When the prior art drug delivery device is used, especially for a long time period, the spring action returning the pawl to its initial position where the pawl engages with the teeth of the toothed wheel of the driving mechanism may become reduced or become insufficient for returning the pawl, resulting in that the audio feedback may be difficult to be distinguished by ear or completely inaudible. Thus, the user may repeatedly inject at least a part of the required drug dose, thereby injecting an excessive drug dose into the patient, or may inject an incomplete drug dose into the patient, resulting in that a required drug dose is not edministered into the patient, since the user may have an incorrect knowledge about a successful or unsuccessful injection of the required drug dose.

Therefore, known drug delivery devices are to be further improved, in particular in order to prevent an excessive or insufficient dose of a drug from being injected into a patient.

Consequently, a technical problem to be solved by the present invention is to develop a drug delivery device which would at least partly eliminate the above disadvantage of the prior art drug delivery device, i.e. eliminate the problem of injection of the excessive or insufficient drug dose into the patient.

### SUMMARY OF THE INVENTION

An objective of the present invention is to develop a drug delivery device solving at least the above-mentioned technical problem.

To achieve the objective of the invention, there is provided a drug delivery device, the device comprising an outer housing; a drug reservoir installed in the outer housing; an actuating mechanism designed to generate an actuating force; a dose-setting mechanism installed at least partly in the outer housing, wherein the dose-setting mechanism is capable of setting a drug dose and functionally connected to the actuating mechanism; an inner housing installed in the outer housing and functionally connected to the dose-setting mechanism; and a rod movably installed at least partly in the inner housing so as to enable the drug to be discharged from the reservoir; wherein the drug delivery device further comprises a driving part provided with radial teeth, wherein the driving part is intermeshed in the outer housing with the inner housing and threadably engaged with rod such that the actuating force is transferred to the rod in order to move the latter depending on the set drug dose; and a cylindrical part provided with at least one resilient element having a paw, wherein the cylindrical part is secured in the outer housing so as to enable the pawl to jump at least over one of the radial teeth of the driving part when rotating the driving part.

A main technical effect provided by the drug delivery device is improved reliability of the drug delivery device when performing the auditory control of the process of injecting the set drug dose by using the drug delivery device, therebe allowing the user, be means of sound signals in the form of clicks or other crack sounds, to focus the user's attention on the beginning, continuation and completion of the process of injecting the set drug dose and to precisely control, by ear, how the above-mentioned steps of the injection process are performed.

Furthermore, a further technical effect provided by the drug delivery device is an extended arsenal of drug delivery technical means using different sound-generating means for notifying the user about the process of injecting the set drug dose.

In yet another embodiment of the present invention, at least one resilient element provided on the cylindrical part may be integral with the cylindrical part.

In another embodiment of the present invention, the cylindrical part may be provided with two resilient elements each having a pawl, wherein the allow one of the pawls may be secured such that the pawls may be arranged around the driving part and angularly shifted by 180 degrees in relation to each other so as to enable both pawls to jump over corresponding radial teeth of the driving part when rotating the driving part.

In yet another embodiment of the present invention, each rotation of the driving part for a predetermined angle corresponding to a single unit of the drug dose may be provided to enable both pawls to jump over corresponding radial teeth of the driving part, so that the above features of the drug delivery device contribute the main technical effect which is improved reliability of the drug delivery device when performing the auditory control of the process of injecting the set drug dose by using the drug delivery device, in particular due to the fact that the user is able to precisely determine or identify, by ear, the fact of delivery of a single unit of the drug dose and, therefore, a total drug dose to be injected into the patient.

In some embodiments of the present invention, the predetermined rotation angle of the driving part may be 18 degrees, and the radial teeth of the driving part may be offset by 18 degrees, and the pawls of the resilient elements may be offset by 180 degrees in relation to each other.

According to one embodiment of the present invention, the pawls may be circumferentially shifted in relation to each other such that each rotation of the driving part for a predetermined angle corresponding to a single unit of the drug dose may enable only one of the pawls to jump on a corresponding one of the radial teeth of the driving part, so that the above features of the drug delivery device contribute the above main technical effect which is improved reliability of the drug delivery device when performing the auditory control of the process of injecting the set drug dose by using the drug delivery device, in particular due to the increased frequency of the sound signals generated in the form of clicks or other crack sounds.

According to another embodiment of the present invention, the driving part may comprise 30 radial teeth, and the predetermined rotation angle of the driving part may be 6 degrees, and the angular shift between the pawls may be 174 degrees.

According to yet another embodiment of the present invention, the driving part may comprise 20 radial teeth, and the predetermined rotation angle of the driving part may be 9 degrees, and the angular shift between the pawls may be 171 degrees.

The provided drug delivery device according to any one of the above-described embodiments of the present invention may be used for administering or injecting drugs into a mammal (for example, a human or an animal), wherein the drug may be selected from a group consisting of insulins, insulin analogs, hormones, heparins, antihistamines, and their derivatives and analogs.

### BRIEF DESCRIPTION OF THE DRAWINGS

FIG. 1 shows a completely assembled drug delivery device according to the present invention having a cap engaged thereon.
FIG. 2 shows the drug delivery device according to the present invention having the cap removed therefrom.
FIG. 3 shows an outer housing used in the drug delivery device of FIG. 1-2.
FIG. 3a shows the outer housing of FIG. 3 as viewed from the distal end thereof.
FIG. 3b shows the outer housing of FIG. 3 as viewed from its proximal end.
FIG. 4 shows the holder for retaining a cartridge used in the drug delivery device of FIG. 1-2.
FIG. 5 shows a cartridge inserted into the holder for retaining a cartridge of FIG. 4.
FIG. 6 shows a dose-setting drum provided with a push button used in the drug delivery device of FIG. 1-2.
FIG. 6a shows the drum of FIG. 6 as viewed from its proximal end, wherein the drum has the push button removed therefrom.
FIG. 6b shows the push button for the drum of FIG. 6a.
FIG. 7 shows the dose-setting drum having a push button mounted thereon and a connecting sleeve engaged thereon.
FIG. 7a shows a connecting sleeve for the drum of FIG. 6-6a.
FIG. 7b shows the connecting sleeve of FIG. 7a as viewed from the distal end thereof.
FIG. 8 shows the drug delivery device according to the present invention having the outer housing of FIG. 3 and 3a-b removed therefrom, the connecting sleeve of FIG. 7a-b removed therefrom and the drum of FIG. 6 and 6a removed therefrom.
FIG. 8a shows a device piece of FIG. 8 having a push button of FIG. 6b further removed therefrom, the holder of FIG. 4 further removed therefrom and the bearing sleeve of FIG. 15a-c further removed therefrom.
FIG. 8b shows the connecting tube used in the drug delivery device of FIG. 1-2.
FIG. 8c shows the connecting tube of FIG. 8b as viewed from its proximal end.
FIG. 8d shows the connecting tube of FIG. 8b as viewed from the distal end thereof.
FIG. 9 shows a threaded screw to the installed in the connecting tube of FIG. 8b-d.
FIG. 10 shows the annular element engaged on the threaded screw of FIG. 9.
FIG. 11 shows a ratchet wheel to be engaged on the connecting tube of FIG. 8b-d.
FIG. 12 shows an inner housing having a rod placed thereinand engaged with the bearing sleeve and the driving part.
FIG. 12a shows a device portion of FIG. 12 having the bearing sleeve further removed therefrom.
FIG. 12b shows the device portion FIG. 12a having the inner housing further removed therefrom.
FIG. 13 shows the rod used in the drug delivery device of FIG. 1-2.
FIG. 14 shows the inner housing used in the drug delivery device of FIG. 1-2.
FIG. 14a shows the inner housing from the distal end thereof.
FIG. 15 shows the inner housing having the rod placed therein and engaged with the bearing sleeve and the driving part as viewed from the distal end thereof.
FIG. 15a shows a bearing sleeve engaged with a retaining ring.
FIG. 15b shows the bearing sleeve of FIG. 15a as viewed from the distal end thereof.
FIG. 15c shows the bearing sleeve of FIG. 15a as viewed from its proximal end having the retaining ring removed therefrom.
FIG. 15d shows a blocking ring to be engaged on the bearing sleeve of FIG. 15c.
FIG. 16 shows the bearing sleeve having a driving part arranged therein from the proximal end of the bearing sleeve.
FIG. 16a shows the driving part as viewed from the distal end thereof.
FIG. 16b shows the driving part as viewed from its proximal end.

### DETAILED DESCRIPTION OF THE INVENTION

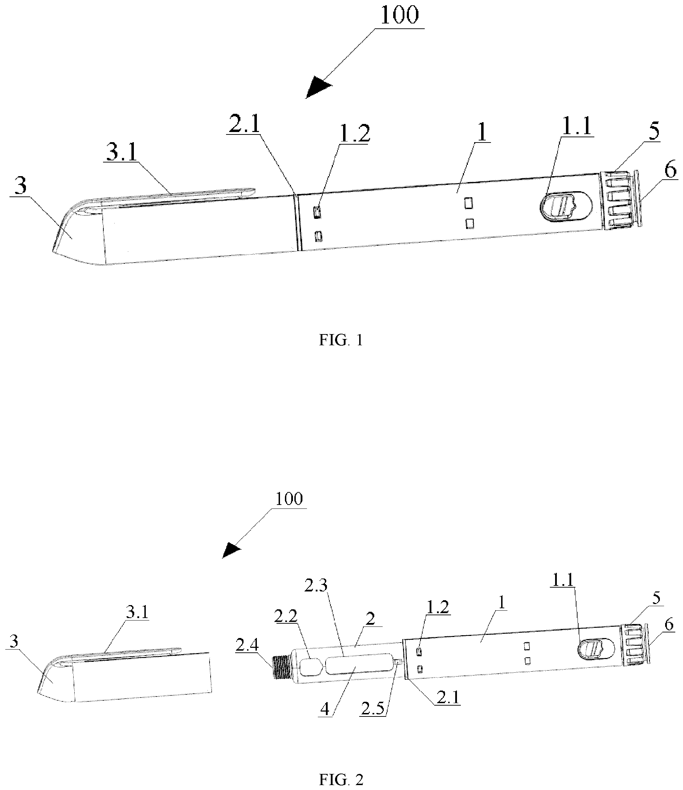

FIG. 1 and 2 shows one embodiment of a drug delivery device 100 according to the present invention used for administering or injecting a drug or medicine in a liquid form (for example, an insulin hormone in case when a patient is a person having diabetes), wherein the drug delivery device 100 is an automatic syringe formed as a pen (hereinafter the syringe pen). It is to note that a user of the syringe pen 100 can be either the patient himself or any other person (e.g., a healthcare professional, a social worker, a relative of the patient, etc.) who may use the syringe pen 100 for administering or injecting the drug into the patient.

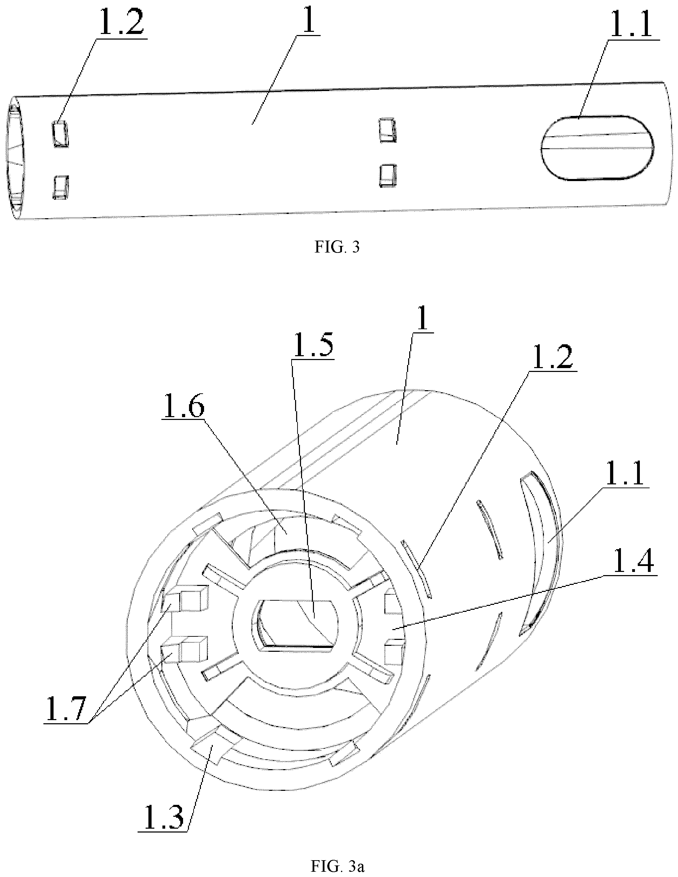

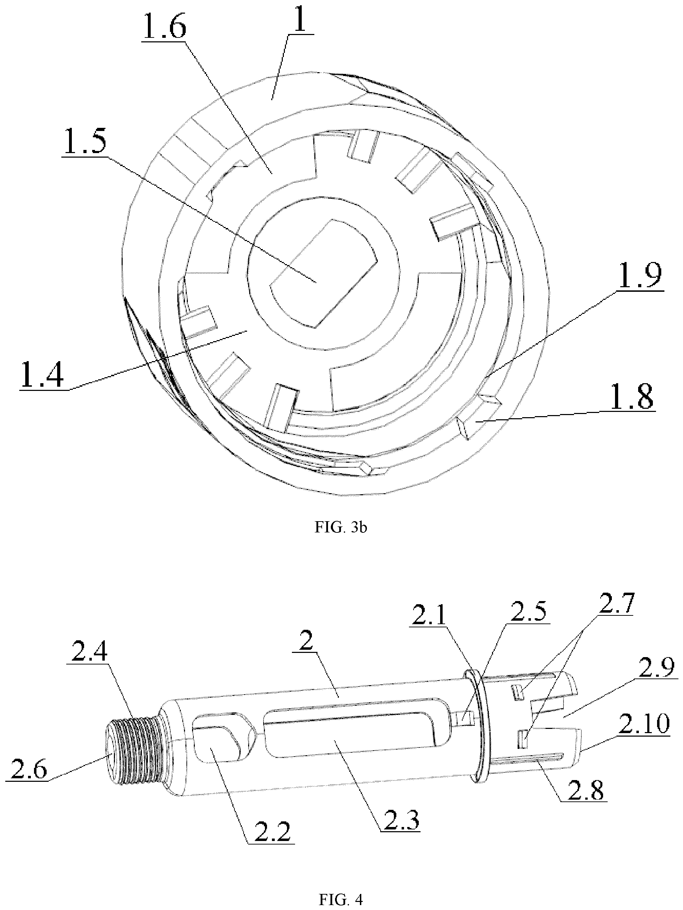

The syringe pen 100 comprises a tubular or cylindrical outer housing 1 of FIG. 3. As shown in FIG. 3a-b, the outer housing 1 has a cylindrical inner cavity which is divided by a partition 1.4 into a proximal section of the outer housing and a distal section of the outer housing, wherein the distal section of the outer housing has a smaller axial length as compared to the proximal section of the outer housing.

As shown in FIG. 1-3 and 3a, the outer housing 1 is further provided at its distal end with fixing means, wherein the fixing means comprise (1) diametrically opposite pairs of fixing slots 1.2 provided in cylindrical wall of the outer housing 1 and spaced from the distal end of the outer housing 1 and the partition 1.4, and (2) diametrically opposite pairs of longitudinal grooves 1.3 provided on the inner surface of the outer housing 1 in the distal section and axially extending from the distal end of the outer housing 1 away from the partition 1.4, and (3) two diametrically opposite blocking protrusions 1.7 designed to engage with corresponding longitudinal cutouts 2.9 of the holder.

As shown in FIG. 3a-b, the partition 1.4 in the outer housing 1 is formed as a disk-shaped or annular element provided with an axial central opening 1.5 and with two diametrically opposite arc-shaped cutouts 1.6, wherein the arc-shaped cutouts 1.6 are provided in the partition 1.4 such that they partly surround the central opening 1.5, wherein the central opening 1.5 of the partition is shaped and sized such that the piston rod 13 runs therethrough, as described below.

As shown in FIG. 3b, the proximal section of the outer housing 1 is also provided with an annular stop protrusion 1.9 provided on the inner surface of the outer housing 1 for a predetermined distance from its proximal end and the partition 1.4, wherein one of the ends of the connecting sleeve 7 abuts on the stop protrusion 1.9, as described below in details. As shown in FIG. 3b, the proximal section of the outer housing 1 is also provided with three locking grooves 1.8 circumferentially provided on the inner surface of the outer housing 1 for a predetermined distance from each other and each extending longitudinally substantially from the proximal end of the outer housing 1 to the stop protrusion 1.9, wherein the locking grooves 1.8 are capable of receiving the fixing ribs 7.1 of the connecting sleeve, as described below in details.

As shown in FIG. 3a and 3b, the proximal end of the outer housing 1 is provided with a viewing window 1.1 allowing the set drug dose to be visually controlled, wherein the proximal end is opposite to the distal end of the of the outer housing 1 as used for inserting the holder 2 thereinto.

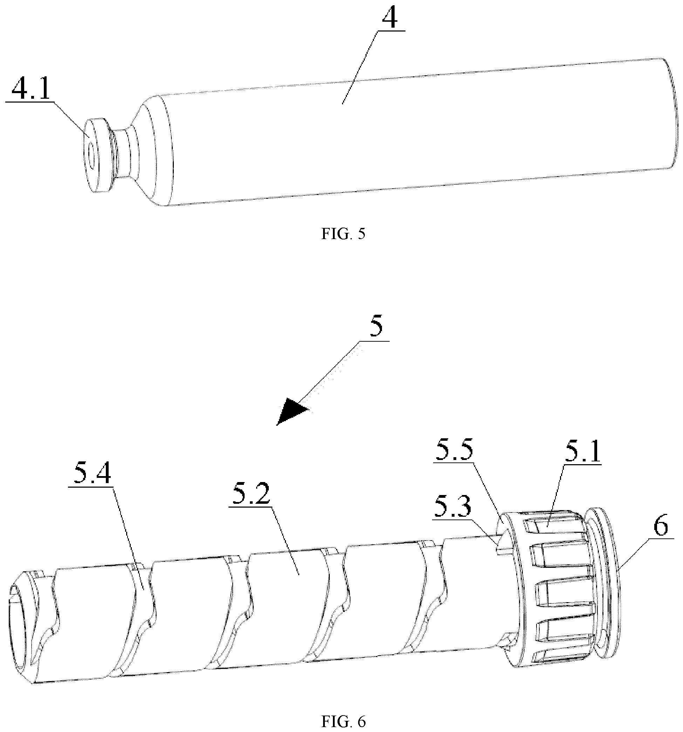

As shown in FIG. 4, the syringe pen 100 also comprises a tubular holder 2 for holding a drug reservoir 4 formed as an ampoule or cartridge (see FIG. 5), wherein the holder 2 is externally divided by an annular lip 2.1 to a holder proximal portion and a holder distal portion. The holder proximal portion is designed to inserted into the distal section of the outer housing 1, and the holder distal portion is designed to be completely covered by a removeable tubular cap 3 of FIG. 1-2. A diameter of the annular lip 2.1 is substantially equal to an external diameter of the outer housing 1 and to an external diameter of the cap 3. It is to note that the outer housing 1 and the holder 2 are designed to be interconnected to form a single protective casing or a single protective envelope, as described below in details.

In one embodiment of the present invention the syringe pen 100 may comprise a housing consisting of a first housing part functioning as the below-described outer housing 1 and a second housing part functioning as the below-described holder 2.

As shown in FIG. 5, the cartridge 4 to be installed or placed in the holder 2 comprises a head 4.1 provided at the distal end of the cartridge 4 and provided with a membrane or partition (not shown), wherein the partition is configured to be pierced to access the drug contained in the cartridge 4 and hermetically seals the cartridge 4 at its distal end. Furthermore, the cartridge 4 is provided with a rubber plunger (not shown) in order to hermetically seal the cartridge 4 at its proximal end, the proximal end being opposite to the distal end of the cartridge 4, and to discharge the drug from the cartridge 4.

The holder 2 of FIG. 4 is provided at its distal end, the distal end of the holder 2 being opposite to the proximal end of the holder 2 as used for inserting the holder 2 into the distal section of the outer housing 1, with a head 2.4. The sleeve with a needle (not shown) may fitted or screwed to the cartridge head 4.1, so that one of the ends of the needle may extend through the central axial opening 2.6 provided in the head 2.4 of the holder and may enter the cartridge 4 inserted into the holder 2, thereby providing communication with the drug contained in the cartridge 4.

As shown in FIG. 1 and 2, the cap 3 is externally provided with a clamp 3.1 for securing the syringe pen 100 to a garment or any another suitable object being accessible to the user.

Furthermore, the holder 2 of FIG. 4 is externally provided with two diametrically opposite retaining protrusions 2.5, each being provided between the lip 2.1 and a corresponding one of the larger viewing windows 2.3. The cap 3, at its proximal end used for engaging the cap 3 on the holder 2, is provided with an annular notch 3.2 provided on the inner surface of the cap 3. It is to note that the cap 3 is used to protect the sleeve with a needle engaged on the cartridge head 4.1 from external influences which would cause, for example, the sleeve damage and/or contamination. Removal of the cap 3 from the holder 2 or taking the cap 3 off the holder 2 allows at least the following operations or actions to be performed: fitting the sleeve with a needle to the cartridge head 4.1, administering or injecting the adjusted or set drug dose into the patient and/or checking the residual amount of the drug in the cartridge 4 by using the below-described viewing windows 2.2, 2.3.

Furthermore, the holder 2 of FIG. 4 is distally provided with two diametrically opposite smaller viewing windows 2.2 and with two diametrically opposite larger viewing windows 2.3, wherein the viewing windows 2.2, 2.3 are provided along the length of the holder 2 between the head 2.4 and the lip 2.1 and axially aligned with each other. When the cap 3 is removed from the second portion of the holder 2, the smaller and larger viewing windows 2.2, 2.3 provide a visual access to different positions of the cartridge 4 inserted into the holder 2, therebe allowing the user to visually evaluate a residual amount of the drug in the cartridge 4.

The holder 2 of FIG. 4 is proximally provided with fixing means provided on the outside of the holder 2, wherein said fixing means comprise two diametrically opposite longitudinal cutouts 2.9 provided such that they divide the proximal end of the holder 2 substantially into two diametrically opposite arc-shaped sectors 2.10, and wherein each of the arc-shaped sectors 2.10 is axially aligned with corresponding smaller viewing window 2.2 and larger viewing window 2.3. The fixing means of the holder 2 also comprise diametrically opposite pairs longitudinal ribs 2.8 extending axially from the lip 2.1 towards the proximal end of the holder 2. The fixing means of the holder 2 also comprise diametrically opposite pairs of latching protrusions 2.7 spaced from the lip 2.1 and the cutouts 2.9.

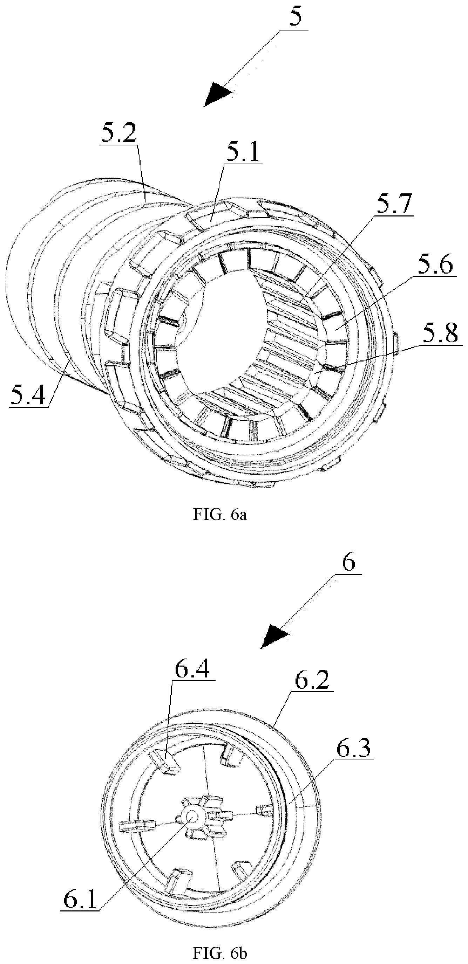

Furthermore, the syringe pen 100 includes a dosing mechanism comprising a rotatable dosing drum 5 (see FIG. 6 and 6a) which comprises a body or main portion 5.2 having a cylindrical inner cavity. The dosing drum 5 also comprises a dose-setting head 5.1 provided at the proximal end of the drum 5 and allowing the dose to be incrementally set when applying a rotational force to the head 5.1, wherein the head 5.1 is integral with the main portion 5.2 of the drum and has a substantially cylindrical inner cavity exposed to the inner cavity of the main portion 5.2 of the drum. The drum head 5.1 is provided with an actuating mechanism in the form of a push button 6 (see FIG. 6b), wherein the push button is provided at the proximal end of the drum 5 and designed to be pressed, in particular the push-button 6 may be pressed with a thumb for initiating the delivery of the set drug dose. Furthermore, the drum head 5.1 may be provided with a dose-setting gripping means (not shown) in the form of axial ribs, wherein the axial ribs may be arranged on an outer surface of the head 5.1 and circumferentially spaced around the head 5.1 in order to facilitate the process of control over the drum head 5.1. As shown in FIG. 6b, the push button 6 has a main portion 6.3, a top 6.2 and an axial pin 6.1 designed to enter the below-described mounting seat 8.10 of the connecting tube 8.

The external diameter of the drum head 5.1 is substantially equal to the external diameter of the outer housing 1 and larger than the external diameter of the main portion 5.2 of the drum, so that the transition from from the proximal end of the main portion 5.2 of the drum to the distal end of the drum head 5.1 externally forms the annular ledge 5.5 of FIG. 6. Furthermore, as shown in FIG. 6, the drum 5 is further provided with two diametrically opposite limiting protrusions 5.3 formed as a tooth and provided on an outer surface of the main portion 5.2 of the drum, wherein each of the limiting protrusions 5.3 extends longitudinally from the annular ledge 5.5 towards the distal end of the drum 5.

As shown in FIG. 6, the main portion 5.2 of the drum is also externally provided with a spiral groove 5.4 extending substantially from the drum head 5.1 to the distal end of the drum 5.1, wherein the distal end of the drum 5.1 is opposite to the proximal end of the drum 5.1. Furthermore, the main portion 5.2 of the drum may be externally provided with a scale (not shown) formed of numerical labels indicative of a number of drug dose units, in particular the drug dose units may be in the range, for example, from 0 to 60 units, wherein an increment may be one unit. The scale may be applied to an outer surface of the main portion 5.2 of the drum between turns of the spiral groove 5.4.

As shown in FIG. 6a, the main portion 5.2 of the drum is also internally provided with ratchet teeth 5.7 formed as identical longitudinal ribs having a trapezoidal cross-section, wherein the ratchet teeth 5.7 are equally spaced on a circumference of the main portion 5.2 of the drum and extend axially from the proximal end of the main portion 5.2 of the drum for a predetermined distance towards the distal end of the main portion 5.2 of the drum, wherein the length of the ratchet teeth 5.7 corresponds at least to a part of the length of the screw 10 or to the length of the screw 10. It is to note that each tooth of the ratchet teeth 5.7 may correspond, for example, to the increment of the set dose for a single unit or to the accumulation of the single dose.

As shown in FIG. 6a, the drum head 5.1 is also provided with pawls 5.6 and an annular cavity 5.8, wherein the pawls 5.6 form a rim consisting of inclined radial teeth circumferentially provided on the inner surface of the head 5.1, and the annular cavity 5.8 is axially provided between the distal end of the drum head 5.1 and the pawls 5.6, so that the annular cavity 5.8 is substantially arranged between the pawls 5.6 and the ratchet teeth 5.7.

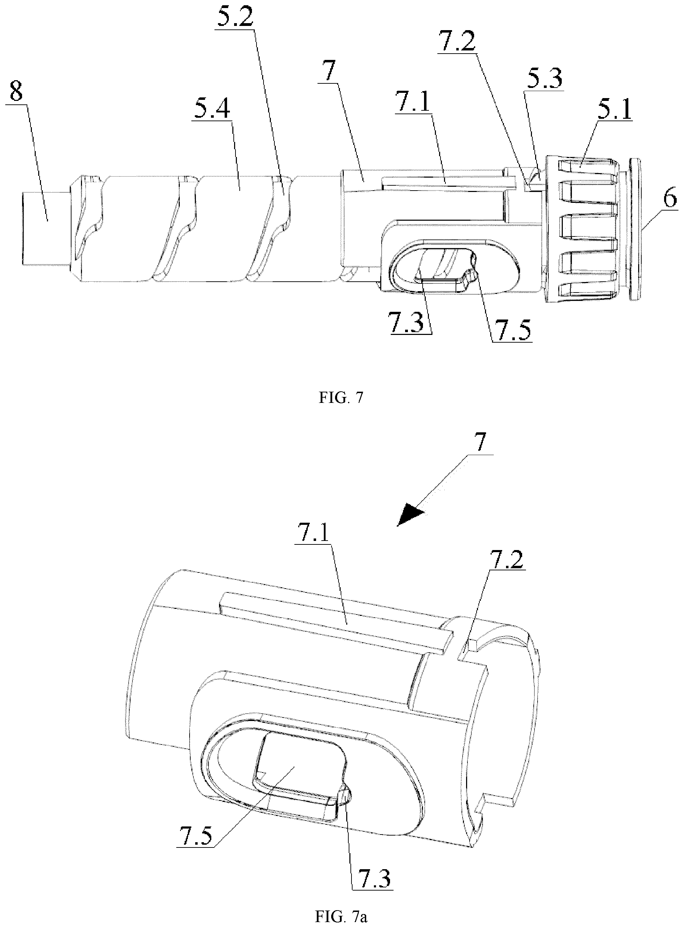

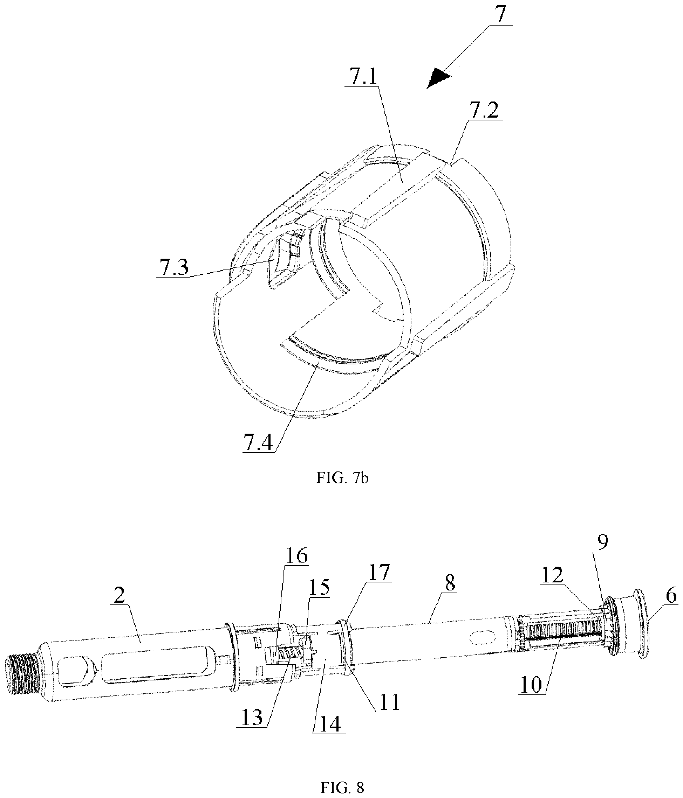

As shown in FIG. 7, the syringe pen 100 also comprises a connecting sleeve 7, wherein the connecting sleeve 7 has a cylindrical inner cavity and is provided with a viewing window 7.3, and dimensions of the viewing window 7.3 are smaller than those of the viewing window 1.1 of the outer housing, and the viewing window 7.3 is provided with a dose indicator 7.5 capable of indicating a number of set units of the drug dose.

As shown in FIG. 7 and 7a-b, the connecting sleeve 7 is also provided with three fixing ribs 7.1, wherein the fixing ribs 7.1 are externally spaced on a circumference of the connecting sleeve 7 and extend axially at least along a portion of the length of the connecting sleeve 7. The connecting sleeve 7 is also provided with two diametrically opposite limiting cutouts 7.2 provided at its proximal end designed for engaging the connecting sleeve 7 on the drum 5 when assembling the syringe pen 100, wherein each of the limiting cutouts 7.2 is used for inserting a corresponding one of the above-described limiting protrusions 5.3 of the drum thereinto. Furthermore, the connecting sleeve 7 is internally provided with a helical rib 7.4 shaped and sized such that the helical rib 7.4 corresponds at least to portion of a turn of the above-described spiral groove 5.4 of the drum.

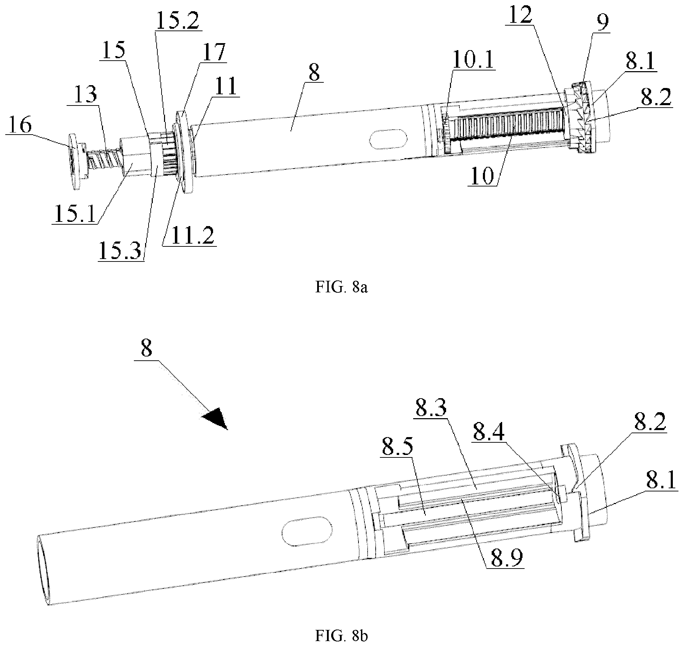

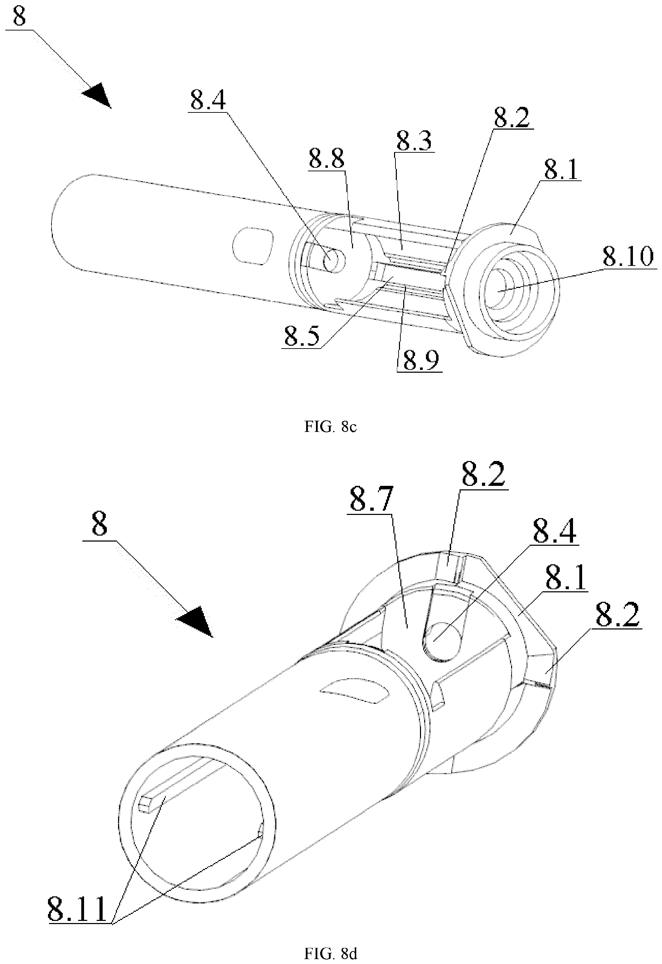

As shown in FIG. 8 and 8a-d, the syringe pen 100 also comprises a connecting tube 8 divided by continuous partitions 8.7 and 8.8 into a proximal compartment, a distal compartment and a central compartment. The proximal compartment is axially defined by the partition 8.7 and the proximal end of the connecting tube 8, wherein the proximal end of the connecting tube 8 is designed for inserting the connecting tube 8 into the drum 5. The distal compartment is axially defined by the partition 8.8 and the distal end of the connecting tube 8, wherein the distal end of the connecting tube 8 is opposite to the proximal end of the connecting tube 8. The central compartment is axially defined by the partition 8.7 and the partition 8.8 and partly exposed to form a cavity 8.3 having a generally cylindrical form. The partitions 8.7 and 8.8 on the side of the cylindrical cavity 8.3 are each provided with a mounting hole 8.4 having a generally circular form, wherein the mounting holes 8.4 are arranged coaxially with each other. As shown in FIG. 8b-c, the cylindrical cavity 8.3 of the connecting tube 8 is also provided with a longitudinal inner protrusion 8.9 provided on the inner surface of the connecting tube 8 and axially extending from the partition 8.7 to the partition 8.8, wherein the inner protrusion 8.9 is provided with a guide recess 8.5 extending axially substantially along an entire length of the inner protrusion 8.9.

As shown in FIG. 8b-c, the connecting tube 8 is provided at its proximal end with a shoulder 8.1 having a generally annular shape, wherein the shoulder 8.1 is provided on the outer surface of the connecting tube 8 such that the shoulder 8.1 is substantially arranged outside the connecting tube 8 between the partition 8.7 of the connecting tube and the proximal end of the connecting tube 8. Furthermore, as shown in FIG. 8b-c, the shoulder 8.1 is provided with four (4) pawls 8.2 designed to mesh with corresponding teeth of the toothed rim 9.2 of the ratchet wheel, wherein the pawls 8.2 are provided on the shoulder 8.1 of the connecting tube from one of its sides such that the pawls 8.2 extend from the shoulder 8.1 of the connecting tube towards the distal end of the connecting tube 8.

As shown in FIG. 8c, the connecting tube 8 is provided at its proximal end with a mounting seat 8.10, wherein the mounting seat 8.10 is shaped and sized such that it corresponds to the axial pin 6.1 of the push button.

As shown in FIG. 8d, the connecting tube 8 is provided at its distal end with two generally identical guide protrusions 8.11, wherein the guide protrusions 8.11 are provided on the inner surface of the connecting tube 8 and substantially face each other. It is to note that the guide protrusions 8.11 are rectilinear, wherein each of the guide protrusions 8.11 extends substantially from the distal end of the connecting tube 8 to the partition 8.8, so that the guide protrusions 8.11 may be engaged with the longitudinal recess 11.3 of the inner housing to allow the rotation or the rotational movement of the connecting tube 8 to be transferred to the inner housing 11, as described below in details.

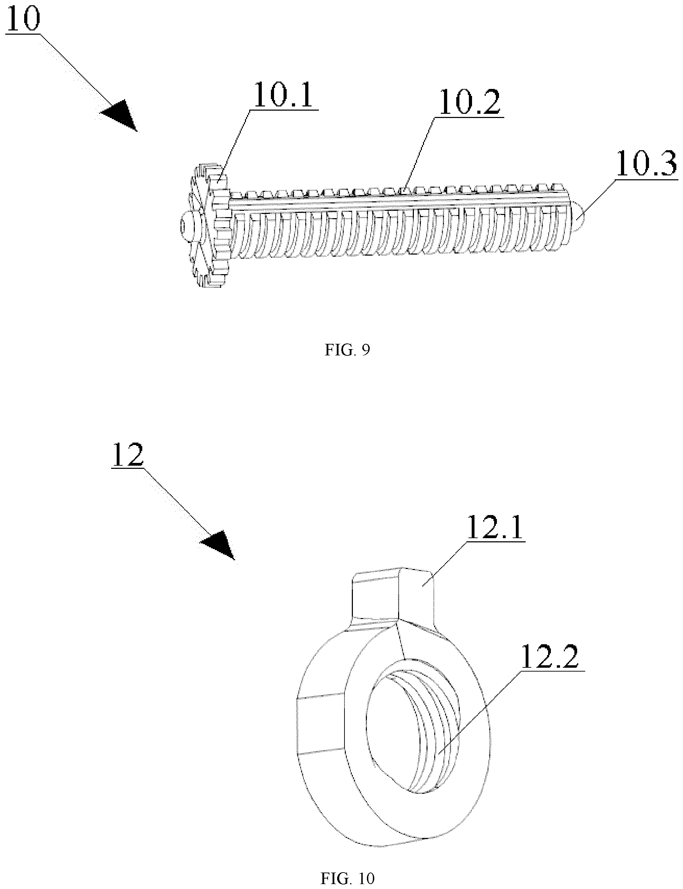

As shown in FIG. 9, the syringe pen 100 also comprises a screw 10 having a leg 10.2 provided with a helical thread 10.2, and a toothed head 10.1, wherein the opposite face ends of the screw 10 are provided with rounded mounting heads 10.3 arranged coaxially with each other.

Furthermore, as shown in FIG. 10, the syringe pen 100 also comprises an annular element 12 externally provided with a radial lug 12.1 and internally provided with a helical rib 12.2, wherein the helical rib 12.2 is shaped and sized such that the helical rib 12.2 corresponds to at least portion of a turn of the helical thread 10.2 of the screw 10.

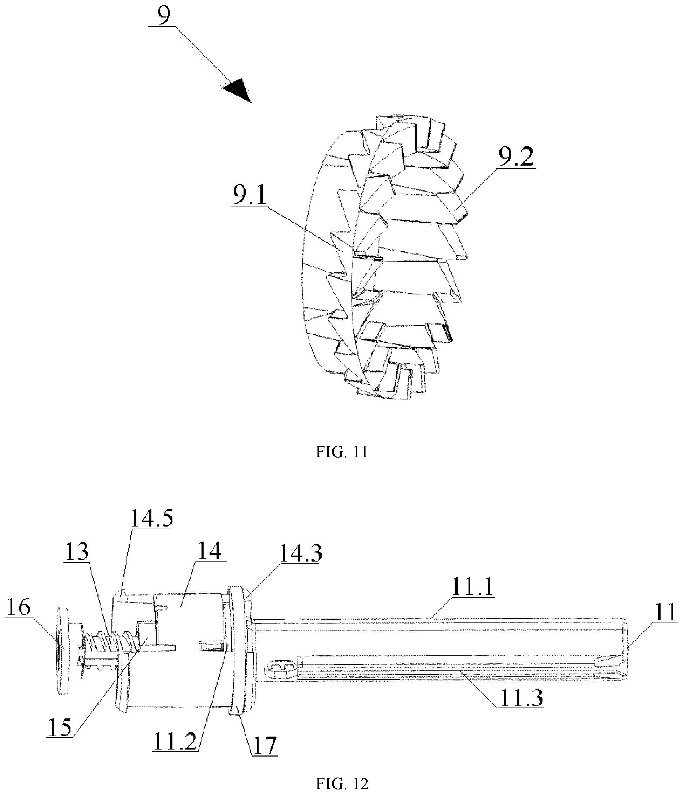

As shown in FIG. 11, the syringe pen 100 also comprises a ratchet wheel 9 provided with a rim 9.1 consisting of inclined axial teeth and with a rim 9.2 consisting of inclined axial teeth, wherein the rims 9.1, 9.2 are formed of identical or generally identical inclined axial teeth. The rims 9.1, 9.2 are interconnected such that they have a shared base, and the inclined axial teeth in the rim 9.1 and corresponding axial teeth in the rim 9.2 face axially towards mutually opposite sides and are generally arranged in a reflectionally symmetric manner and radially offset in relation to each other.

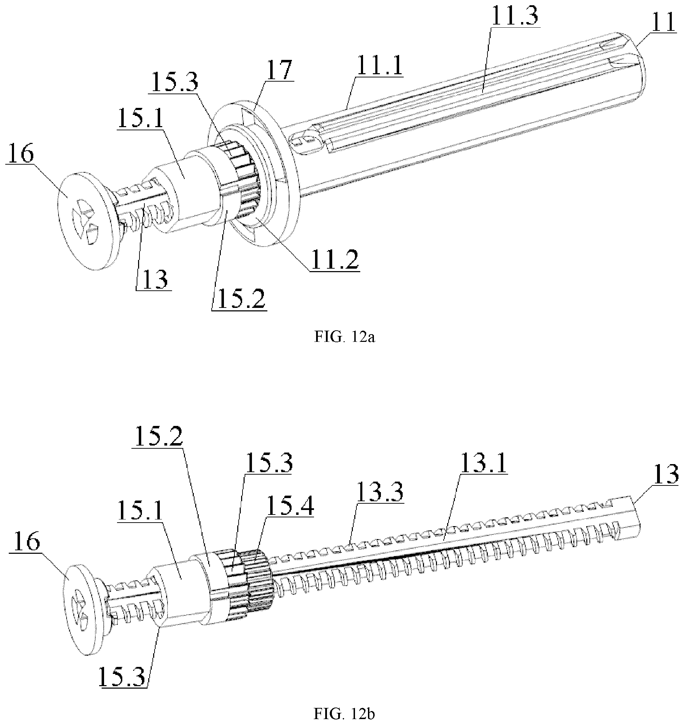

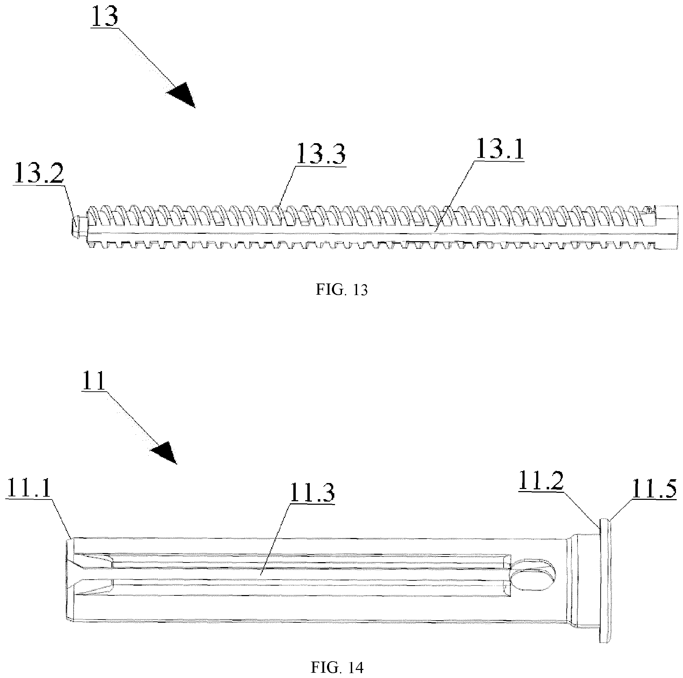

As shown in FIG. 12 and 12a, the syringe pen 100 also comprises a cylindrical inner housing 11 formed as a hollow tube or tubular part and consisting of a main portion or body 11.1. As shown in FIG. 12a, the below-described rod 13 of FIG. 12b and 13 is arranged or placed in the inner housing 11.

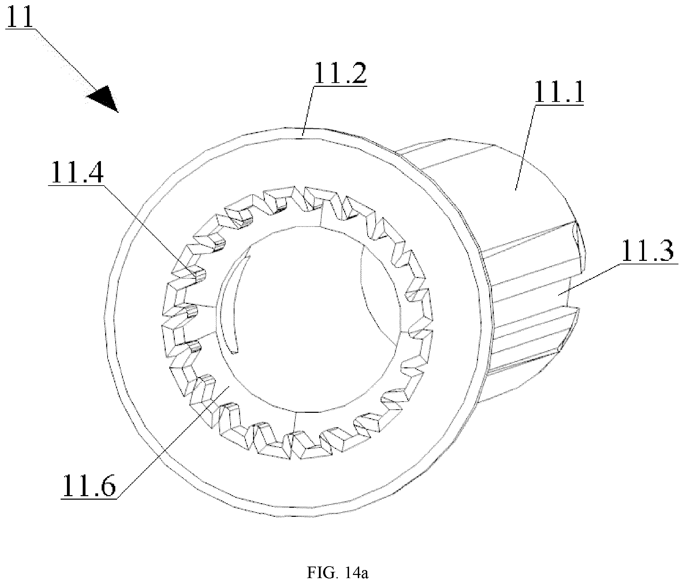

As shown in FIG. 14, the body 11.1 of the inner housing is thickened at the distal end of the inner housing 11, wherein the distal end of the inner housing 11 is opposite to the proximal end of the inner housing 11 used for fitting the connecting tube 8 thereto or engaging the connecting tube 8 thereon. The body 11.1 is externally provided with an annular retaining lip 11.2 provided at the distal end of the inner housing 11 such that it surrounds the thickened portion of the body 11.1, wherein the retaining lip 11 is substantially integral with the body 11.1 of the inner housing. It is to note that the thickened portion of the body 11.1 has an external diameter which is larger than that of the remaining portion of the body 11.1, the remaining portion extending from the thickened portion to the proximal end of the inner housing 11, but smaller than the external diameter of the retaining lip 11.2, so that the transition between the thickened portion of the body 11.1 and the retaining lip 11 substantially forms an annular limiting protrusion 11.5 from the outside of the inner housing 11 of FIG. 14. It is to further note that the thickened portion of the body 11.1 has an inner diameter which is larger than that of the remaining portion of the body 11.1, so that the transition between a portion of the body 11.1 having a smaller inner diameter and the thickened portion of the body 11.1 having a larger inner diameter substantially forms an annular stop ledge 11.6 from the inside of the inner housing 11 of FIG. 14a.

Furthermore, as shown in FIG. 14 and 14a, the body 11.1 of the inner housing is externally provided with a rectilinear groove or rectilinear longitudinal recess 11.3 of the inner housing, wherein the longitudinal recess 11.3 extends from the proximal end of the inner housing 11 substantially to the thickened portion of the body 11.1 of the inner housing.

Furthermore, as shown in FIG. 14a, the distal end of the inner housing 11 is provided with inclined radial teeth 11.4, wherein the inclined radial teeth 11.4 are equally circumferentially spaced on the inner surface of the thickened portion of the base 11.1 of the inner housing, and each of the inclined radial teeth 11.4 extends substantially from the distal end to the stop ledge 11.6, i.e. have an elongated shape. It is to note that the inclined radial teeth 11.4 are shaped and sized such that they engage or mesh with the below-described inclined radial teeth of the connecting toothed rim 15.4 of the driving part 15, as described below in details.

Furthermore, as shown in FIG. 12, 12a-b, 13 and 15, the syringe pen 100 comprises a piston rod 13 formed as a rod and externally provided with a helical thread 13.3, wherein the helical thread 13.3 is provided substantially along an entire length of the piston rod 13 and designed to engage with the helical thread 15.5 of the driving part to form a threaded connection, wherein the portion of the helical thread 13.3 is axially cut out or removed from two diametrically opposite sides of the piston rod 13 substantially along an entire length thereof, thereby forming two diametrically opposite flat surfaces 13.1. Thus, the piston rod 13 is shaped and sized such that the piston rod 13 is allowed to extend through the central opening 1.5 provided in the partition 1.4 of the outer housing 1, thereby preventing the piston rod 13 from being rotated or blocking the rotation of the piston rod 13.

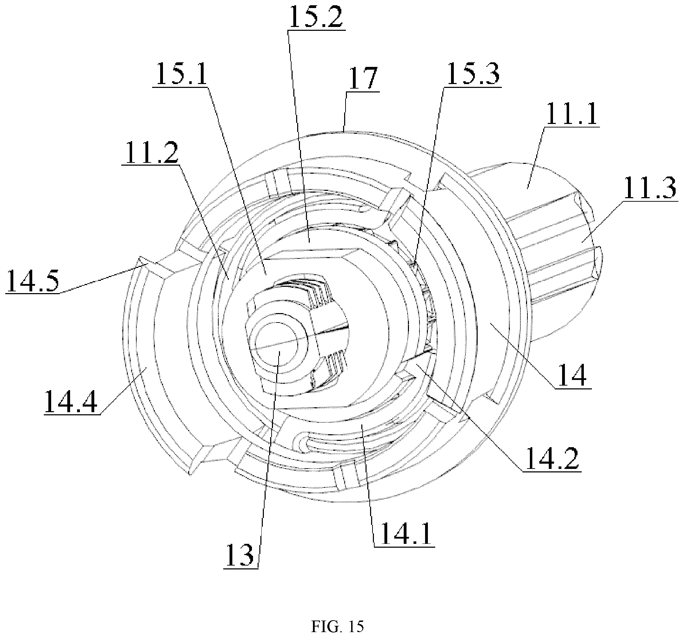

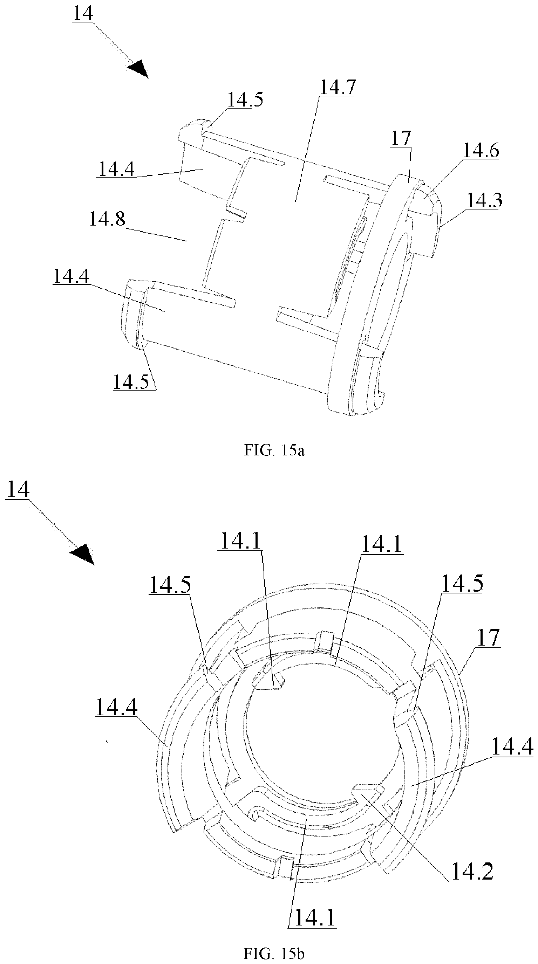

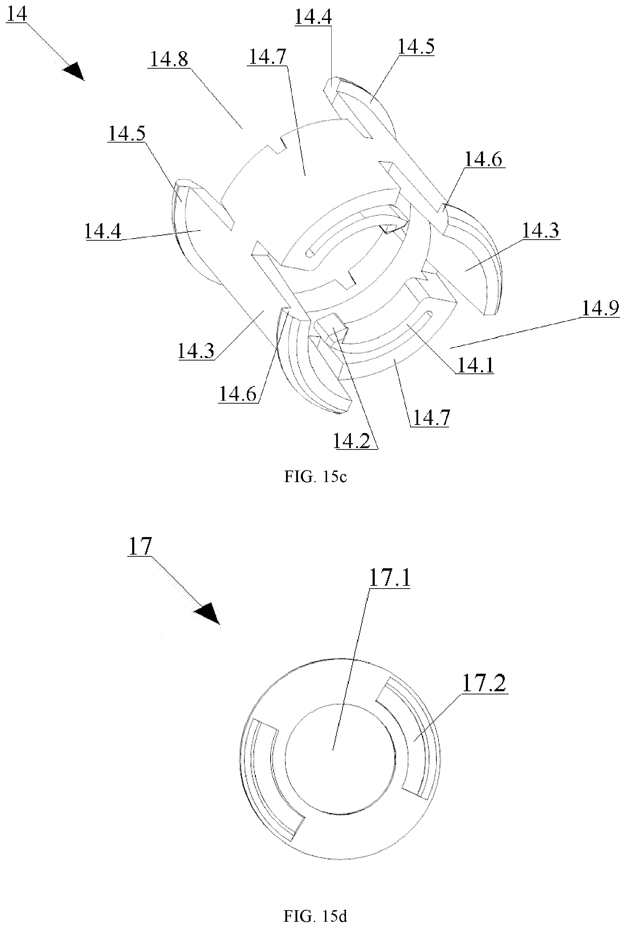

Furthermore, as shown in FIG. 13 and 15, the distal end of the piston rod 13 is provided with a mounting head 13.2 used for fitting the disk-shaped element 16 of FIG. 12 and 12a-12b thereto or engaging the disk-shaped element 16 of FIG. 12 and 12a-12b thereon, wherein the disk-shaped element 16 is designed to engage with a plunger of the cartridge 4 so as to allow the drug dose to be discharged from the cartridge 4 in a required amount, in particular in a volume corresponding to the set drug dose to be delivered to an injection site used for injecting the dose into the patient in a particular case of use of the syringe pen 100.

As shown in FIG. 12, 15, 15a-c and 16, the syringe pen 100 also comprises a bearing sleeve 14 formed as a cylindrical part and having a substantially cylindrical inner cavity.

Furthermore, as shown in FIG. 15, 15a and 15c, the bearing sleeve 14 has two diametrically opposite axial cutouts 14.8 provided at the distal end of the bearing sleeve 14, wherein the distal end of the bearing sleeve 14 is opposite to the proximal end thereof used to intermesh the bearing sleeve 14 with the retaining ring 17 so as to envelop or surround an external portion of the driving part 15, and the external portion extends outside the inner housing 11 when inserting the driving part 15 into the inner housing 11. The axial cutouts 14.8 are arranged such that they divide the distal end of the bearing sleeve 14 substantially into two diametrically opposite arc-shaped segments 14.4, wherein the arc-shaped segments 14.4 are shaped and sized such that they are allowed to be inserted into corresponding arc-shaped cutouts 1.6 of the partition, and each of the arc-shaped segments 14.4 is provided with an arc-shaped hook 14.5 designed to mesh with the partition 1.4 of the outer housing.

Furthermore, as shown in FIG. 15 and 15a-c, the bearing sleeve 14 has two diametrically opposite axial cutouts 14.9 provided at the proximal end of the bearing sleeve 14, wherein axial cutouts 14.9 are arranged such that they divide the proximal end of the bearing sleeve 14 substantially into two diametrically opposite arc-shaped segments 14.3, and the arc-shaped segments 14.3 are shaped and sized such that they are allowed to be inserted into corresponding arc-shaped openings 17.2 of the retaining ring, and each of the arc-shaped segments 14.3 is provided with an arc-shaped hook 14.6 designed to mesh with the retaining ring.

Furthermore, as shown in FIG. 15a and 15c, each of the arc-shaped segments 14.4 is integral with a corresponding one of the arc-shaped segments 14.3 so as to form a corresponding one of two diametrically opposite elongated arc-shaped portions of the bearing sleeve 14, wherein the elongated arc-shaped portions are interconnected by two shortened arc-shaped portions 14.7 of the bearing sleeve so as to form an integral part having a cylindrical shape, wherein each of the shortened arc-shaped portions 14.7 is formed by producing a corresponding one of the axial cutouts 14.8 at the distal end of the bearing sleeve 14 and corresponding one of the axial cutouts 14.9 at the proximal end of the bearing sleeve 14.

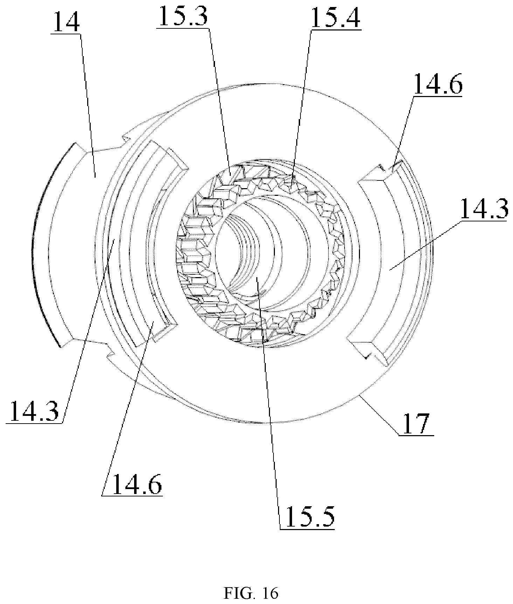

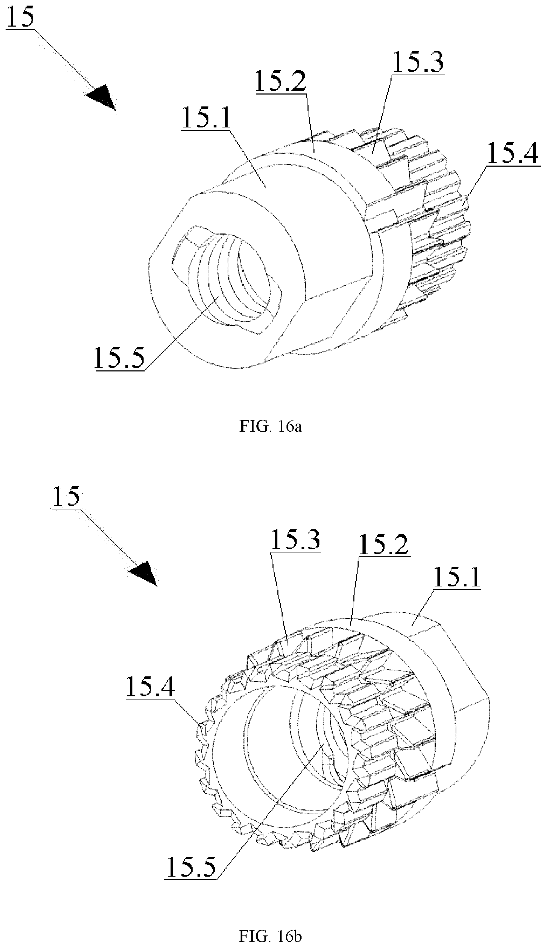

As shown in FIG. 15, 15a-b, 15d and 16, the syringe pen 100 also comprises a retaining ring 17 having a central opening 17.1, wherein the central opening 17.1 is shaped and sized such that the retaining ring 17 may be fitted to or engaged on the thickened portion of the body 11.1 of the inner housing. The retaining ring 17 is provided with two generally identical arc-shaped openings 17.2 designed to be reflectionally symmetric and surrounding the central opening 17.1 from the diametrically opposite sides, wherein the arc-shaped openings 17.2 are shaped and sized such that corresponding elongated arc-shaped protrusions 14.3 are allowed to be inserted into arc-shaped openings 17.2, and the arc-shaped hooks 14.6 are allowed to mesh with the retaining ring 17. It is to further note that the retaining ring 17 is configured to be fitted to or engaged on the thickened portion of the body 11.1 of the inner housing from the proximal end of the inner housing 11, so that the retaining ring 17 is fitted to the body 11.1 of the inner housing and the retaining ring 17 and is adjacent to or abuts on the retaining lip 11.2. Therefore, when the elongated arc-shaped protrusions 14.3 of the bearing sleeve are inserted into corresponding arc-shaped openings 17.2 of the retaining ring such that the arc-shaped hooks 14.6 of the bearing sleeve are allowed to mesh with the retaining ring 17 and when the arc-shaped segments 14.4 of the bearing sleeve are inserted into corresponding arc-shaped cutouts 1.6 of the partition of the outer housing such that the arc-shaped hooks 14.5 are allowed to mesh with the partition 1.4 of the outer housing, the bearing sleeve 14 on one side is fixedly releasably secured to the retaining ring 17 such that the retaining ring 17 is adjacent to or abuts on the retaining lip 11.2 of the inner housing providing a slit therebetween, and the bearing sleeve 14 on the other side is fixedly releasably secured to the outer housing 1, thereby allowing the bearing sleeve 14 to be retained in a required position in relation to the below-described driving part 15, in particular in relation to the outer toothed rim 15.3 of the driving part. Thus, the above prevents the bearing sleeve 14 and the retaining ring 17 from being axially moved and rotated in relation to the driving part 15, however the driving part 15 itself, including the outer toothed rim 15.3 of the driving part, is still capable of rotating together with the inner housing 11 in relation to the bearing sleeve 14 and to the retaining ring 17 secured to the bearing sleeve 14.

As shown in FIG. 15 and 15b-c, the bearing sleeve 14 is also internally provided with two generally identical diametrically opposite spring or resilient elements 14.1, wherein each of resilient elements 14.1 is formed as a curved or bent elongated plate element, lobe or plate spring and faced toward the inner cavity of the bearing sleeve 14. As shown in FIG. 15 and 15b-c, each of the resilient elements 14.1 of the bearing sleeve is curved or bent such that said resilient element 14.1 has a base being integral with an inner surface of the bearing sleeve 14, wherein the base extends from the inner surface for a predetermined distance. Also, each of the resilient elements 14.1 has an arc-shaped portion spaced from the inner surface of the bearing sleeve 14, wherein one of the ends of the arc-shaped portion is integrally connected to the base, wherein the other end of the arc-shaped portion is provided with a hook or a paw 14.2 having a tip oriented or faced towardwards the inner cylindrical cavity of the bearing sleeve 14. Therefore, the resilient elements 14.1 of the bearing sleeve are integral with the bearing sleeve 14 and generally spaced from the inner surface of the bearing sleeve 14, wherein their pawls 14.2 are oriented in a required manner within the inner cylindrical cavity of the bearing sleeve 14, so that each of tips of the pawls 14.2 may be engaged or meshed with a corresponding one of the radial teeth of the outer toothed rim 15.3 of the driving part. The tips of the pawls 14.2 of the resilient elements as provided on the inner surface of the bearing sleeve 14 are arranged circumferentially and angularly shifted or offset by one hundred eighty (180) degrees in relation to each other and face each other within the inner cylindrical cavity of the bearing sleeve 14. It is to note that two resilient elements 14.1 having the pawls 14.2 and forming a functional pair of the resilient elements of the bearing sleeve 14 and the outer toothed rim 15.3 of the driving part having the inclined radial teeth engaged with the pawls 14.2 substantially form a ratchet mechanism in the lower or distal portion of the syringe pen 100, wherein the ratchet mechanism allows audible sounds in the form of clicks or other crack sounds to be generated or produced, thereby providing a sound feedback for the process of delivering the set drug dose to a dose injection site used for injecting the dose into the patient.

As shown in FIG. 16 and 16a-b, syringe pen 100 also comprises a cylindrical driving part 15 having a generally cylindrical inner cavity. The driving part 15 is externally provided with an annular protuberance 15.2 which divides the driving part 15 into an end portion 15.1 and a toothed portion. The end portion 15.1 axially extends from the annular protuberance 15.2 to the distal end of the driving part 15, wherein the distal end of the driving part 15 is opposite to the proximal end of the driving part 15, the proximal end being used for inserting the driving part 15 into the inner housing 11. The toothed portion axially extends from the annular protuberance 15.2 to the proximal end of the driving part 15, wherein the toothed portion consists of an outer toothed rim 15.3 and a connecting toothed rim 15.4 of the driving part. The outer toothed rim 15.3 has inclined radial teeth designed to engage with the tips of the pawls 14.2 of the resilient elements so as to allow audiable sounds in the form of clicks or other crack sounds to be generated or produced. The connecting toothed rim 15.4 of the driving part has radial teeth designed to engaged with the radial teeth 11.4 of the inner housing to form a toothed connection. It is to note that the outer toothed rim 15.3 has a radius which is larger than that of the connecting toothed rim 15.4 and which generally corresponds to an external radius of the annular protuberance 15.2, and the inclined radial teeth of the connecting toothed rim 15.4 have a larger axial extension as compared to the inclined radial teeth of the outer toothed rim 15.3, i.e. the inclined radial teeth of the connecting toothed rim 15.4 are designed as elongated. It is to further note that the outer toothed rim 15.3 comprises twenty (20) of the inclined radial teeth provided on the outer surface of the threaded portion of the driving part 15 and offset or shifted along the external circumference of the threaded portion for eighteen (18) degrees in relation to each other. Therefore, each of the twenty inclined radial teeth of the outer toothed rim 15.3 of the driving part is arranged each eighteen (18) degrees, and the offset or shift of one hundred eighty (180) degrees between two pawls 14.2 substantially corresponds to ten (10) radial teeth of the outer toothed rim 15.3 of the driving part.

Therefore, in the mode of delivering the set drug dose to a dose injection site used for injecting the dose into the patient, rotation of the driving part 15 in relation to the bearing sleeve 14, the bearing sleeve 14 and the resilient elements 14.1 provide on the inner surface of the bearing sleeve 14 having a fixed position in relation to the outer toothed rim 15.3 of the driving part, allows at least one of the two pawls 14.2 to jump over a corresponding one of the inclined radial teeth of the outer toothed rim 15.3 of the driving part, thereby generating or producing sounds in the form of audible clicks or other audible crack sounds. In particular, in the mode of delivering the set drug dose to a dose injection site used for injecting the dose into the patient, the rotation of the driving part 15 for each eighteen (18) degrees substantially results in that each of the two pawls 14.2 simultaneously jump over a corresponding one of the inclined radial teeth of the outer toothed rim 15.3 of the driving part, the inclined radial teeth being offset or shifted on the external circumference of the toothed portion of the driving part 15 for eighteen (18) degrees in relation to each other. In other words, when the inner housing 11 and the driving part 15 are rotated together for eighteen (18) degrees, both pawls 14.2 will be each able to simultaneously jump over a corresponding one of the inclined radial teeth of the outer toothed rim 15.3 of the driving part, thereby generating an audible sound in the form of a click or another crack sound having an increased volume. Thus, such resulting sound having an increased volume is formed of two separate sounds overlapped with each other, wherein each of the overlapped sounds is generated when one of two pawls 14.2 jumps over a corresponding one of twenty (20) inclined radial teeth of the outer toothed rim 15.3 of the driving part. In view of the above explanations, the resulting sound may be better distinguished by the user, especially in cases when the user has different hearing problems, when external distractions are presented or any external noises are presented, so that such user may more precisely control, by ear, the process of delivering the set drug dose to a dose injection site used for injecting the dose into the patient. Therefore, the pawls 14.2 are shifted or offset circumferentially in relation to the inclined radial teeth of the outer toothed rim 15.3 of the driving part such that each eighteen (18) degrees the engagement of both pawls 14.2 with two inclined radial teeth of the outer toothed rim 15.3 of the driving part, each of the inclined radial teeth of the outer toothed rim 15.3 corresponding to one of two pawls 14.2, results in the generation or production of a resulting sound in the form of a click or crack, the resulting sound having an increased volume, when rotating the inner housing 11 together with the driving part 15 in one direction, namely in a direction corresponding to the mode of delivering the set drug dose to a dose injection site used for injecting the dose into the patient. Otherwise, when the inner housing 11 is rotated in an opposite direction corresponding to a mode of accumulating or setting the drug dose to be delivered to an injection site used for injecting the dose into the patient, the above-described engagement of both pawls 14.2 with two inclined radial teeth of the outer toothed rim 15.3 of the driving part allows the further rotation or rotational movement of the inner housing 11 and the driving part 15 to be blocked.

In one embodiment of the present invention, the bearing sleeve 14 may be provided with three resilient elements, wherein each of the resilient elements may be similar to any of the resilient elements 14.1. Therefore, the bearing sleeve 14 may have three (3) pawls, wherein each of the pawls may be similar to any one of the pawls 14.2, and tips of the pawls may be circumferentially arranged and angularly shifted or offset by one hundred twenty (120) degrees in relation to each other. The outer toothed rim 15.3 of the driving part may comprise twenty (20) inclined radial teeth which may be shifted or offset on the external circumference of the toothed portion of the driving part 15 for eighteen (18) degrees in relation to each other. In other words, each of the inclined radial teeth of the outer toothed rim 15.3 may be arranged each eighteen (18) degrees, so that the offset by one hundred twenty (120) degrees between each pair of adjacent pawls 14.2 of the three pawls of the bearing sleeve 14 may substantially correspond to six and two-thirds (6 and 2/3) of inclined radial teeth of the outer toothed rim 15.3 of the driving part, and the rotation of the driving part 15 together with the inner housing 11 for eighteen (18) degrees allows each of the three pawls 14.2 to jump only once over a corresponding one of twenty inclined radial teeth of the outer toothed rim 15.3 of the driving part. Therefore, in the present embodiment of the present invention, the pawls 14.2 are shifted or offset circumferentially in relation to the inclined radial teeth of the outer toothed rim 15.3 of the driving part such that each six (6) degrees the engagement between one of the three pawls 14.2 and a corresponding one of the inclined radial teeth of the outer toothed rim 15.3 results in the generation or production of a sound in the form of a click or crack when rotating the inner housing 11 together with the driving part 15 in one direction, namely in a direction corresponding to the mode of delivering the set drug dose to a dose injection site used for injecting the dose into the patient. Otherwise, when the inner housing 11 is rotated in an opposite direction corresponding to the mode of accumulating or setting the drug dose to be delivered to an injection site used for injecting the dose into the patient, the above-described engagement between one of the three pawls 14.2 and a corresponding one of the inclined radial teeth of the outer toothed rim 15.3 allows the further rotation or rotational movement of the inner housing 11 and the driving part 15 to be blocked. In the present embodiment of the present invention, the above-described features of the circumferential shift or offset 14.2 in relation to the inclined radial teeth of the outer toothed rim 15.3 of the driving part allow the user to more precisely control, by ear, the process of delivering the set drug dose to a dose injection site used for injecting the dose into the patient due to the increased frequency of feedback sounds in the form of clicks or cracks, the feedback sounds accompanying the process of delivering the set drug dose.

In another embodiment of the present invention, the bearing sleeve 14 may be provided with three resilient elements, wherein each of the resilient elements may be similar to any of the resilient elements 14.1. Therefore, the bearing sleeve 14 may have three (3) pawls, wherein each of the pawls may be similar to any one of the pawls 14.2, and tips of the pawls may be circumferentially arranged and angularly shifted or offset by one hundred twenty (120) degrees in relation to each other. The outer toothed rim 15.3 of the driving part may comprise forty (40) inclined radial teeth which may be shifted or offset on the external circumference of the toothed portion of the driving part 15 for nine (9) degrees in relation to each other. In other words, each of the inclined radial teeth of the outer toothed rim 15.3 may be arranged each nine (9) degrees, so that the offset by one hundred twenty (120) degrees between each pair of adjacent pawls 14.2 of the three pawls of the bearing sleeve 14 may substantially correspond to thirteen and one-third (13 and 1/3) of the inclined radial teeth of the outer toothed rim 15.3 of the driving part, and the rotation of the driving part 15 together with the inner housing 11 for nine (9) degrees allows each of the three pawls 14.2 to jump only once over a corresponding one of the forty inclined radial teeth of the outer toothed rim 15.3 of the driving part. Therefore, in the present embodiment of the present invention, the pawls 14.2 are shifted or offset circumferentially in relation to the inclined radial teeth of the outer toothed rim 15.3 of the driving part such that each three (3) degrees the engagement between one of the three pawls 14.2 and a corresponding one of the inclined radial teeth of the outer toothed rim 15.3 results in the generation or production of a sound in the form of a click or crack when rotating the inner housing 11 together with the driving part 15 in one direction, namely in a direction corresponding to the mode of delivering the set drug dose to a dose injection site used for injecting the dose into the patient. Otherwise, when the inner housing 11 is rotated in an opposite direction corresponding to the mode of accumulating or setting the drug dose to be delivered to an injection site used for injecting the dose into the patient, the above-described engagement between one of the three pawls 14.2 and a corresponding one of the inclined radial teeth of the outer toothed rim 15.3 allows the further rotation of the inner housing 11 and the driving part 15 to be blocked. In the present embodiment of the present invention, the above-described features of the circumferential shift or offset 14.2 in relation to the inclined radial teeth of the outer toothed rim 15.3 of the driving part allow the user to more precisely control, by ear, the process of delivering the set drug dose to a dose injection site used for injecting the dose into the patient due to the increased frequency of feedback sounds in the form of clicks or cracks, the feedback sounds accompanying the process of delivering the set drug dose.

In yet another embodiment of the present invention, the bearing sleeve 14 may be provided with three resilient elements, wherein each of the resilient elements may be similar to any of the resilient elements 14.1. Therefore, the bearing sleeve 14 may have three (3) pawls, wherein each of the pawls may be similar to any one of the pawls 14.2, and tips of the pawls may be circumferentially arranged and angularly shifted or offset by one hundred twenty (120) degrees in relation to each other. The outer toothed rim 15.3 of the driving part may comprise thirty (30) inclined radial teeth which may be shifted or offset on the external circumference of the toothed portion of the driving part 15 for twelve (12) degrees in relation to each other. In other words, each of the inclined radial teeth of the outer toothed rim 15.3 may be arranged each twelve (12) degrees, so that the offset by one hundred twenty (120) degrees between each pair of adjacent pawls 14.2 of the three pawls of the bearing sleeve 14 may substantially correspond to ten (10) inclined radial teeth of the outer toothed rim 15.3 of the driving part, and the rotation of the driving part 15 together with the inner housing 11 for twelve (12) degrees allows all the three pawls 14.2 to substantially simultaneously each jump over a corresponding one of the thirty (30) inclined radial teeth of the outer toothed rim 15.3 of the driving part. Therefore, in the present embodiment of the present invention, the pawls 14.2 are shifted or offset circumferentially in relation to the inclined radial teeth of the outer toothed rim 15.3 of the driving part such that each twelve (12) degrees the engagement between the three pawls 14.2 and three inclined radial teeth of the outer toothed rim 15.3 of the driving part, each corresponding to one of the three pawls 14.2, results in the generation or production of a resulting sound in the form of a click or crack sound having an increased volume. Thus, such resulting sound is formed of three separate sounds overlapped with each other, wherein each of the overlapped sounds is generated when one of the three pawls 14.2 jumps over a corresponding one of the thirty (30) inclined radial teeth of the outer toothed rim 15.3 of the driving part when the inner housing 11 is rotated together with the driving part 15 in one direction, namely in a direction corresponding to the mode of delivering the set drug dose to a dose injection site used for injecting the dose into the patient. Otherwise, when the inner housing 11 is rotated in an opposite direction corresponding to the mode of accumulating or setting the drug dose to be delivered to an injection site used for injecting the dose into the patient, the above-described engagement between the three pawls 14.2 and three inclined radial teeth of the outer toothed rim 15.3 of the driving part allows the further rotation of the inner housing 11 and the driving part 15 to be blocked.

In one embodiment of the present invention, the resilient elements 14.1 of the driving part, each being provided with the paw 14.2, may be internally secured to the bearing sleeve 14 or attached thereto such that their pawls 14.2 may be arranged in a desired manner and oriented in the inner cavity of the bearing sleeve 14, as described above in details. In yet another embodiment of the present invention, the resilient elements 14.1, each being provided with the pawl 14.2, may be each inserted into a special throughbore or special throughbores made or provided in the housing of the bearing sleeve 14, and secured therein such that their pawls 14.2 are arranged in a desired manner and oriented in the inner cavity of the bearing sleeve 14, as described above in details.

According to one embodiment of the present invention, the bearing sleeve 14 may be provided with at least one resilient element 14.1 having a paw 14.2, wherein the resilient element 14.1 is arranged and oriented in the inner cavity of the bearing sleeve 14 such that the resilient element 14.1 engages with the inclined radial teeth of the outer toothed rim 15.3 of the driving part and jumps over a corresponding one of the inclined radial teeth during each rotation or rotational movement of the inner housing 11 for a predetermined rotation angle corresponding to a single drug dose to be delivered to the drug injection site.

When assembling the syringe pen 100, the distal end of the driving part 15 is partly inserted into the inner housing 11 such that the face end of the driving part 15 from its proximal end abuts on the annular stop ledge 11.6 of the inner housing, and the inclined radial teeth of the connecting toothed rim 15.4 of the driving part are engaged with the radial teeth 11.4 of the inner housing so as to form a tight toothed connection therebetween, thereby allowing the rotation or rotational movement of the driving part 15 when rotating or rotatably moving the inner housing 11 having the driving part 15 inserted thereinto, in particular allowing the inner housing 11 and the driving part 15 to rotate together substantially with the same rotational speed. It is to note that in case when the driving part 15 is partly inserted into the inner housing 11, the outer toothed rim 15.3 of the driving part, the annular protuberance 15.2 of the driving part and the end portion 15.1 of the driving part are arranged outside the inner housing 11 and axially extend away from the distal end of the inner housing 11. Then, when assembling the syringe pen 100, the retaining ring 17 is fitted to or engaged on the inner housing 11 having the driving part 15 inserted thereinto from the proximal end of the inner housing 11, such that the retaining ring 17 abuts on the retaining lip 11.2.

Then, when assembling the syringe pen 100, the bearing sleeve 14 with two resilient elements 14.2 is engaged on the inner housing 11 from the distal end of the inner housing 11, the distal end of the inner housing 11 being designed such that the external portion of the driving part 15 projects therefrom, so as to allow the arc-shaped segments 14.3 of the bearing sleeve to be inserted into corresponding arc-shaped openings 17.2 of the retaining ring and the arc-shaped hooks 14.6 to be engaged with the retaining ring 17, resulting in that the bearing sleeve 14 substantially completely externally surrounds the driving part 15 and provides the required position thereof in relation to the external portion the driving part 15, and the pawls 14.2 of the resilient elements are engaged with corresponding inclined radial teeth of the outer toothed rim 15.3 of the driving part in a desired manner, wherein the bearing sleeve 14 is not capable of axially moving away from the retaining lip 11.2 of the inner housing.

Then, the piston rod 13 is inserted into the driving part 15 from the proximal end of the driving part 15, the driving part 15 being inserted, by means of the toothed connecting rim 15.4, into the inner housing 11 having the bearing sleeve 14 engaged thereon; then, the helical thread 13.3 of the rod is engaged with the helical thread 15.5 of the driving part, in particular by axially screwing the distal end of the piston rod 13 to the driving part 15 from its proximal end, so that the portion of the piston rod 13 provided with the head 13.2 protrudes or extends outside the distal end of the driving part 15, and another portion of the piston rod 13 extends in the interior space of the driving part 15, and the remaining larger portion of the piston rod 13 extends in the interior space of the inner housing 11, as shown, for example, in FIG. 12a-b.

Then, the inner housing 11, the inner housing 11 being provided with the bearing sleeve 14 engaged on the inner housing 11 in the above-described manner and provided with the driving part 15 inserted into the inner housing 11 in the above-described manner and having the piston rod 13 screwed thereto in the above-described manner, is installed in or inserted into the outer housing 1 from its proximal end such that the end face of the driving part 15 as viewed from the distal end thereof abuts on the partition 1.4 of the outer housing from the proximal section of the outer housing 1, and the projecting portion of the piston rod 13 extends through the central opening 1.5 of the partition of the outer housing, partly extends outside the partition 1.4 of the outer housing and enters the distal section of the outer housing 1. Furthermore, when performing the above-described insertion of the inner housing 11 into the outer housing 1, the bearing sleeve 14 enters, by means of the arc-shaped portions14.5, the arc-shaped cutouts 1.6 of the partition 1.4 such that the arc-shaped hooks 14.5 mesh with the partition 1.4 of the outer housing 1 from its distal section, thereby allowing the bearing sleeve 14 to be fixedly secured to the outer housing 1, preventing the bearing sleeve 14 from being rotated in relation to the outer housing 1 and the inner housing 11 and further preventing the bearing sleeve from being axially moved in relation to the inner housing 11 and to the outer housing 1. Thus, the above results in the fixation of a required position of the bearing sleeve 14 in relation to the driving part 15, in particular in relation to the inclined radial teeth of the outer toothed rim 15.3 of the driving part. It is to note that the step of assembling the syringe pen 100 results in that the bearing sleeve 14 is arranged coaxially between the inner housing 11 and the outer housing 1, and the inner housing 11 is not allowed to axially or reciprocately move in relation to the outer housing 1. It is to further note that the insertion of the rod 13 into the central opening 1.5 provided in the partition 1.4 of the outer housing substantially prevents the piston rod 13 from being rotated or rotationally moved about its axis in relation to the driving part 15 and/or the inner housing 11 or blocks said rotation of the piston rod 13.

Then, when assembling the syringe pen 100, the distal end of the connecting sleeve 7 is inserted into the outer housing 1 from the distal end of the outer housing 1 such that the end face of the connecting sleeve 7 as viewed from the distal end thereof substantially abuts on the stop protrusion 1.9 of the outer housing, and the fixing ribs 7.1 of the sleeve frictionally engage with the locking grooves 1.8 of the outer housing such that the connecting sleeve 7 is secured in relation to the outer housing 1 in a required orientation. When the connecting sleeve 7 is secured in relation to the outer housing 1 in the required orientation, the viewing window 7.3 is aligned with the viewing window 1.1 such that the dose indicator 7.5 and the drug dose mark indicated by the dose indicator 7.5 are visible through the viewing window 1.1 of the outer housing. It is to note that the insertion of the connecting sleeve 7 into the outer housing 1 results in that the connecting sleeve 7 is arranged coaxially with the outer housing 1.

When further assembling the syringe pen 100, the drum 5 is inserted into the connecting sleeve 7 installed in the above-described manner in the outer housing 1, in particular by providing the helical engagement between the spiral groove 5.4 of the drum and the helical rib 7.4 of the connecting sleeve and placing each of the limiting protrusions 5.3 of the drum in a corresponding one of two limiting cutouts 7.2 of the connecting sleeve, so that the end face of the connecting sleeve 7 from its proximal end is faced toward the annular ledge 5.5 of the drum and spaced from the annular ledge 5.5 of the drum so as to form an annular slot therebetween. It is to note that in the below-described mode of delivering the set drug dose to the dose injection site, in case when the user presses the button 6 or applies a generally axial pressing force to the button 6 to allow the drum 5 to be completely pushed into the outer housing 1 or completely returned back from the outer housing 1, the limiting protrusions 5.3 of the drum may abut on the connecting sleeve 7 within the limiting cutouts 7.2 of the connecting sleeve to prevent the drum 5 from being rotated away from a zero position (initial position) of the drum 5, the zero position corresponding to the delivery of the entire drug dose accumulated or set by the user, and, therefore, preventing an excessive drug amount exceeding the accumulated or set drug dose to be delivered to the dose injection site to be injected into the patient. In other words, the drum 5 is installed in the connecting sleeve 7 by screwing the distal end of the drum 5 into the connecting sleeve 7 from its proximal end. It is to note that the screwing of the drum 5 to the connecting sleeve 7 results in that the drum is arranged coaxially with the connecting sleeve 7, and the connecting sleeve 7 is arranged concentrically between the outer housing 1 and the drum 5, and the dose indicator 7.5 indicates a dose mark which may be seen through the viewing window 7.3. Also, when the drum 5 is screwed to the connecting sleeve 7, the end face of the outer housing 1 from its proximal end is faced toward the annular ledge 5.5 of the drum and spaced from the annular ledge 5.5 of the drum so as to form an annular slot therebetween, however the end face of the outer housing 1 does not abut on the annular ledge 5.5 of the drum due to the limiting cutouts 7.2 of the connecting sleeve and the limiting protrusions 5.3 of the drum that provide the annular slot between the connecting sleeve 7 and the annular ledge 5.5 of the drum. Thus, the drum head 5.1 is arranged outside the outer housing 1 and substantially abuts on the proximal end of the outer housing 1. It is to note that when assembling the syringe pen, a dose mark indicated by the dose indicator 7.5 may be changed depending on a particular assembling stage. However, when the syringe pen 100 is completely assembled, the dose indicator 7.5 indicates a zero dose mark, wherein the zero dose mark may be seen through the viewing window 7.3 when the syringe pen 100 is completely assembled.

Therefore, the locking grooves 1.8 of the outer housing and the fixing ribs 7.1 of the connecting sleeve, the locking grooves 1.8 and the fixing ribs 7.1 being engaged with each other, in combination with the stop protrusion 1.9 of the outer housing and the annular ledge 5.5 of the drum, the stop protrusion 1.9 and the annular ledge 5.5 of the drum being used by corresponding face ends of the connecting sleeve 7 for abutting thereon, prevent the connecting sleeve 7 from being rotated and axially moved in relation to the outer housing 1 and to the drum 5.

Then, when assembling the syringe pen 100, the annular element 12 is screwed to the threaded screw 10 such that the annular element 12 is arranged at the end of the threaded screw 10, the end being opposite to the toothed head 10.1 of the screw, wherein said position of the annular element 12 corresponds to an initial position of the annular element 12 on the toothed screw 10, the initial position indicating that the process of delivering the set drug dose to the dose injection site used for injecting the dose into the patient is not still performed by the user. Furthermore, when assembling the syringe pen 100, the toothed screw 10 having the annular element 12 screwed to the screw 10 in the above-described manner is rotatably installed in cylindrical cavity 8.3 of the connecting tube such that the mounting heads 10.3 of the screw enter corresponding mounting holes 8.4 of the connecting tube, and the radial lug 12.1 of the annular element enters the guide recess 8.5 of the connecting tube, so that the rotation of the screw 10 in the cylindrical cavity 8.3 allows the annular element 12 to axially straightforwardly move along the length of the toothed screw 10 from the toothed head 10.1 of the screw to the partition 8.7 of the connecting tube, wherein the position of the annular element 12 on the threaded screw 10 will substantially correspond to the drug dose consumed or delivered to the dose injection site when using the syringe pen 100. Furthermore, when assembling the syringe pen 100, the ratchet wheel 9 is fitted to or engaged on the connecting tube 8 such that the ratchet wheel 9 is arranged coaxially with the connecting tube 8, and the pawls 8.2 of the connecting tube mesh with corresponding teeth of the toothed rim 9.2 of the ratchet wheel.

Then, when assembling the syringe pen 100, the connecting tube 8 having the ratchet wheel 9 fitted thereto tube 8 in the above-described manner and provided with the screw 10, the screw 10 being provided with the annular element 12 screwed thereto and being installed in the cylindrical cavity 8.3 of the connecting tube in the above-described manner, is installed or inserted into the drum 5 from its proximal end such that the guide protrusions 8.11 of the connecting tube are engaged with the longitudinal recesses 11.3 of the inner housing, so that the longitudinal recesses substantially function as guides for the guide protrusions 8.11 of the connecting tube, thereby allowing the body 11.1 of the inner housing to be pushed or advanced into the connecting tube 8 from its distal end such that the face end of the connecting tube 8 substantially abuts on the retaining ring 17 as viewed from the distal end of the connecting tube 8. The above-described assembling operation results in that the connecting tube 8 is arranged concentrically between the inner housing 11 and the drum 5. When the drum 5 is rotated in the direction of setting the drug dose (in particular, when decreasing or increasing the drug dose to be delivered to the injection site used for injecting the dose into the patient), the connecting tube 8 is capable of axially moving in relation to the outer housing 1 and to the inner housing 11 due to the movement of the guiding protrusions 8.11 of the connecting tube along the longitudinal recesses 11.3 of the inner housing and not capable of allowing the rotation of the inner housing 11. When the drum 5 is rotated in the direction of delivering the set drug dose, the connecting tube 8 is capable of tranferring all the rotational moment or at least a portion thereof from the drum 5 to the inner housing 11 due to the tight and reliable protrusion-recess type connection between the inner housing 11 and the connecting tube 8, the connection being formed as a result of the engagement between the longitudinal recesses 11.3 of the inner housing and corresponding guide protrusions 8.11 of the connecting tube, resulting in the rotation of the inner housing 11 jointly or together with the connecting tube 8.

Furthermore, when the connecting tube 8 is inserted by its distal end into the drum 5, the shoulder 8.1 of the proximal end of the connecting tube 8 together with the ratchet wheel 9 are completely positioned in the drum head 5.1, wherein the annular portion of the ratchet wheel 9, the annular portion being not externally provided wtih the toothed rims 9.1, 9.2 having the inclined axial teeth, is arranged in the annular cavity 5.8 of the drum head, and the annular face end of the annular portion of the ratchet wheel 9 is arranged adjacent to face ends of the ratchet teeth 5.7 of the drum at the proximal end of the main portion 5.2 of the drum. Furthermore, when the connecting tube 8 is inserted by its distal end into the drum 5, the teeth of the toothed head 10.1 of the screw are engaged with the ratchet teeth 5.7 of the drum, thereby allowing the toothed screw 10 to rotate about its axis, wherein the connection formed of the guide recess 8.5 of the connecting tube and the radial lug 12.1 inserted into the guide recess 8.5 prevents the annular element 12 from being rotated about the threaded screw 10 when transferring the rotational moment from the drum 5 to the screw 10. Therefore, the rotation of the drum 5 in the mode of setting the drug dose, in particular in the clockwise direction corresponding to the increase of the drug dose to be delivered to the dose injection site, or in the counter-clockwise direction corresponding to the decrease of the drug dose to be delivered to the dose injection site, allows the pawls 5.6 of the drum to engage with the teeth of the toothed rim 9.1 and allows the inclined axial teeth of the toothed rim 9.2 to engage with the pawls 8.2 of the connecting tube such that the rotational movement of the drum 5 is converted to an axial movement of the connecting tube 8 in relation to the inner housing 11 away from the retaining lip 11.2 of the inner housing, and the rotation of the drum 5 in the mode of delivering the set drug dose, in particular in the counter-clockwise direction, allows the pawls 5.6 of the drum to engage with the inclined axial teeth of the toothed rim 9.1 and allows the inclined axial teeth of the toothed rim 9.2 to engage with the pawls 8.2 of the connecting tube such that the rotational moment is transferred from the drum 5 to the connecting tube 8 and, then, from the connecting tube 8 to the inner housing 11, resulting in a coupled rotation of the drum 5, the connecting tube 8 and the inner housing 11 in the same direction, i.e. in the counter-clockwise direction.

Then, when assembling the syringe pen 100, a spring (not shown) is installed or inserted into the mounting seat 8.10 of the connecting tube from the proximal end of the drum 5, so that the spring at one of its ends is supported by a bottom of the mounting seat 8.10. Then, the button 6 is inserted into the proximal end of the connecting tube 8 such that the main portion 6.3 of the button and the axial pin 6.1 of the button enter the mounting seat 8.10, and the top 6.2 of the button becomes arranged outside the drum head 5.1, wherein the spring (not shown) inserted into the mounting seat 8.10 becomes fitted to or engaged on the axial pin 6.1 of the button, and the other end of the spring abuts on a corresponding one of horizontal ledges of the axial pin 6.1 of the button, thereby allowing button 6 to be spring-loaded and, thus, allowing the button 6 to return to its initial position when the button 6 is released. It is to note that the spring-loaded button 6 arranged in the connecting tube 8 from the proximal end of the connecting tube 8, and the ratchet mechanism of the syringe pen 100 as formed of the teeth of the toothed rim 9.2 of the ratchet wheel and the pawls 8.2 of the connecting tube, each pawl 8,2 being intermeshed with a corresponding one of the teeth of the toothed rim 9.2, prevent the drum 5 from being unintentionally rotated and, accordingly, prevent the drug dose from being unintentionally accumulated or set.

Then, when assembling the syringe pen 100, the disk-shaped element 16 is fitted to or engaged on the head 13.2 of the piston rod, the head 13.2 being provided at the distal end of the piston rod 13.

Then, when assembling the syringe pen 100, the cartridge 4 is inserted into the holder 2 from its proximal end such that the cartridge head 4.1 is arranged in the inner cavity of the holder head 2.4.

Then, when assembling the syringe pen 100, the holder 2 having the cartridge 4 inserted thereinto is inserted by the proximal portion of the holder 2 into the distal section of the outer housing 1, so that the longitudinal ribs 2.8 of the holder frictionally engage with corresponding longitudinal grooves 1.3 of the outer housing, the face ends of the sectors 2.10 of the holder from the proximal end of the holder 2 at least partly abut on the partition 1.4 of the outer housing, and the latching protrusions 2.7 of the holder latch behind the fixing slots 1.2 of the outer housing, and the blocking protrusions 1.7 of the outer housing are inserted such that they abut on corresponding longitudinal cutouts 2.9 of the holder, thereby allowing the proximal portion of the holder 2 to be press-fitted in the distal section of the outer housing 1 such that the face end of the outer housing 1 from the distal end thereof abuts on the lip 2.1 and allowing the holder 2 to be secured in relation to the outer housing 1 in a predetermined orientation where corresponding smaller viewing window 2.2 and larger viewing window 2.3 are axially aligned with the viewing window 1.1. Therefore, when the holder 2 is inserted into the outer housing 1, the above-described fixing means of the holder 2 engage with the above-described fixing means of the outer housing 1, thereby preventing the holder 2 from being rotated and axially moved in relation to the outer housing 1. Furthermore, when the holder 2 is inserted into the outer housing 1, the disk-shaped element 16 engages with a plunger of the cartridge 4 such that the drug is discharged from the cartridge 4 in a required amount, in particular in an amount corresponding to the set drug dose to be delivered to the dose injection site used for injecting the dose into the patient.

In a final step of assembling the syringe pen 100, the cap 3 may be fitted to or engaged on the holder 2 from the distal end thereof, so that the retaining protrusions 2.5 may frictionally engage with the annular notch 3.2 so as to allow the cap 3 to be press-fitted on the distal portion of the holder 2 such that the face end of the cap 3 from its proximal end abuts on the annular lip 2.1. To check an amount of the drug, replace the sleeve with the needle (not shown), initially fit the sleeve with the needle (not shown) to the cartridge head 4.1 and/or inject the drug remaining in the cartridge 4, if required, the cap 3 may be removed from or taken off the holder 2 by applying a corresponding force thereto in a direction which is generally opposite to a direction of engaging the cap 3 on the holder 2, wherein the applied force allows the annular notch 3.2 of the cap to be released from the frictional engagement with the retaining protrusions 2.5 of the holder.

When the completely assembled syringe pen 100 is initially used, the user is required to remove the cap 3 from the holder 2 and fit the sleeve with the needle (not shown) to the cartridge head 4.1 or engage the sleeve with the needle (not shown) on the cartridge head 4.1.

To define or set the required drug dose, the user is required to grip the drum head 5.1 with fingers of the hand, in particular to grip the gripping means of the drum head, if any, and to apply a rotary force to the drum head 5.1 in the clockwise direction, the rotary force being sufficient for rotating the drum 5 in the clockwise direction, wherein as the drum 5 is rotated, the drum 5 is moved out the outer housing 1 in a step-by-step manner, resulting in that the drum head 5.1 provided with the push button 6 will move away from the proximal end of the outer housing 1. Furthermore, when the drum 5 is rotated in the clockwise direction, the drum 5 will transfer, by means of the connection as formed by the ratchet teeth 5.7 of the drum and the teeth of the toothed head 10.1 of the screw that are engaged with the ratchet teeth 5.7, the rotational moment from the drum 5 to the screw 10, resulting in the rotation of the threaded screw 10 about its axis in the same direction so as to allow the annular element 12 to be axially moved along the length of the threaded screw 10 to the toothed head 10.1 of the screw towars the partition 8.8 of the connecting tube. When the rotation of the drum 5 is stopped by the user, a position of the annular element 12 on the threaded screw 10 will substantially corresponds to the drug dose initially set or accumulated by the user and initially delivered to the dose injection site. However, if the syringe pen 100 is further used, a position of the annular element 12 on the threaded screw 10 after stopping the rotation of the drum 5 will substantially correspond to a sum of totally consumed volume of the drug (i.e. a volume of the drug consumed by the user or delivered to the drug injection site for performing all the injections during the whole time of usage of the syringe pen before the current process of setting the required drug dose as performed by the user) and a current volume of the drug, the current volume corresponding to a currently set drug dose to be delivered to the dose injection site. Furthermore, when the drum 5 is rotated in the clockwise direction, the user may observe in the viewing window 1.1 one of the scale marks, the observed mark being indicated by the dose indicator 7.5 of the connecting sleeve and corresponding substantially to a number of drug dose units set or accumulated by the user. The drum 5 advanced from the outer housing 1 during rotation of the drum 5 in the direction of setting the drug dose will substantially pull the connecting tube 8 in an advancement direction (as provided due to the above-described toothed connections between the drum 5, the ratchet wheel 9 and the connecting tube 8), thereby allowing the connecting tube 8 to be axially moved in relation to the inner housing 11 away from the retaining lip 11.2 of the inner housing due to the above-described protrusion-recess type connection between the connecting tube 8 and the inner housing 11, in particular due to the axial movement or displacement of the guide protrusions 8.11 of the connecting tube along the longitudinal recesses 11.3 of the inner housing in the longitudinal direction away from the retaining lip 11.2 of the inner housing when pulling the connecting tube 8 by means of the drum 5.

To deliver a required drug dose to the dose administration or injection site, the user using the syringe pen 100 presses the outer surfaces of the top 6.2 of the button or applies a generally axial pressing force thereto during a time period, thereby pressing the button 6 and, thus, compressing the spring (not shown), the spring being used for biasing the button 6 in the mounting seat 8.10. Pressing the button 6 allows the force applied by the user to be at least partly transferred to the connecting tube 8, thereby allowing the connecting tube 8 to rotate together with the drum 5 in the counter-clockwise direction, i.e. in the direction of delivering the set drug dose, in particular due to the above-described toothed connections between the drum 5, the ratchet wheel 9 and the connecting tube 8. Due to the above-described protrusion-recess type connection between the connecting tube 8 and the inner housing 11, the connecting tube 8 transfers the rotational moment to the inner housing 11, resulting in rotation of the inner housing 11 in the counter-clockwise direction, i.e. in the direction of delivering the set drug dose. Due to the above-described toothed connection between the inner housing 11 and the driving part 15, the inner housing 11 in turn transfers the rotational moment to the driving part 15, resulting in the rotation of the driving part 15 in the counter-clockwise direction, i.e. in the direction of delivering the set drug dose. Then, due to the above-described threaded connection between the driving part 15 and the piston rod 13 and due to the above-described means for blocking the rotation of the piston rod 13, the rotation of the driving part 15 about its axis in turn results in the axial displacement or movement of the piston rod 13, the piston rod 13 being provided at its distal end with the disk-shaped element 16, so as to have the drug discharged from the cartridge 4 due to to the pushing force applied by the disk-shaped element 16 to the plunger (not shown) of the cartridge 4 from the proximal end of the cartridge 4. In other words, the driving part 15 in the syringe pen 100 functions as a driving mechanism. Due to the functional engagement between the driving mechanism and the inner housing 11, the driving mechanism finally allows the rotation of the drum 5, the rotation of the drum 5 being transferried by the connecting tube 8 to the inner housing 11, to be transformed to an axial movement of the piston rod 13, in particular due to the above-described functional connections provided between the drug-dosing drum 5, the connecting tube 8, the inner housing 11 and the piston rod 13. Therefore, the push button 6 allows the actuating force to be produced, wherein the produced actuating force is then transferred, by means of the drum 5 functionally connected to the connecting tube 8, to the connecting tube 8, and then transferred to the inner housing 11 from the connecting tube 8 functionally connected to the inner housing 11, and then transferred to the driving part 15 from the inner housing 11 functionally connected to the driving part 15, and finally transferred to the piston rod 13 from the driving part 15 functionally connected to the piston rod 13 so as to displace or move the piston rod 13 away from the proximal end of the inner housing 11, wherein an amount of the axial displacement or movement of the piston rod 13 substantially corresponds to the drug dose set or accumulated by the user. Finally, when the drum 5 is rotated in the direction of delivering the set drug dose, the axial movement of the piston rod 13 results in that the plunger (not shown) of the cartridge 4 is advanced, by means of the disk-shaped element 16, towards the distal end of the cartridge 4 such that the drug discharged from the cartridge 4 is supplied to the dose administration or injection site, wherein an amount of the movement of the piston rod 13 and, thus, an amount of advancement of the plunger of the cartridge 4 by the piston rod 13 solely depend on the volume of the drug dose in the dose units, the drug dose being previously set by the user by using the drum 5, as described above.

It is to note that the axial movement of the piston rod 13 as caused by rotation of the drum 5 in the direction of delivering the set drug dose results, in particular, in the rotation of the driving part 15 as caused by transferring, via the connecting tube 8, the rotational moment from the drum 5 to the inner housing 11, together with the inner housing 11 and, therefore, each of the two pawls 14.2 of the resilient elements of the bearing sleeve simultaneously engages at least with a corresponding one of the inclined radial teeth of the outer toothed rim 15.3 of the driving part depending on a volume of the set or accumulated drug dose to be delivered to the dose injection site, thereby allowing each of the two pawls 14.2 to jump at least once over at least a corresponding one of the inclined radial teeth of the outer toothed rim 15.3 of the driving part. Thus, a number of jumps performed by each of the two pawls 14.2 over the inclined radial teeth of the outer toothed rim 15.3 of the driving part or a number of the inclined radial teeth of the outer toothed rim 15.3 of the driving part, the inclined radial teeth being used by each of the two pawls 14.2 for jumping thereover, depends on the rotation angle of the driving part 15. Also, the rotation angle of the driving part 15 in turn depends primarly on the volume of the set or accumulated drug dose to be delivered to the dose injection site. In other words, when the driving part 15 is rotated, each jump of both pawls 14.2 over corresponding inclined radial teeth of the outer toothed rim 15.3 of the driving part takes place in parallel or simultaneously, thereby allowing production or generation of a resulting sound signal or a sound in the form of a click or crack sound having an increased volume as formed of two separate sounds overlapped with each other, wherein each of the overlapped sounds is generated when one of the two pawls 14.2 jumps over a corresponding one of twenty (20) inclined radial teeth of the outer toothed rim 15.3 of the driving part.

For example, in case when the driving part 15 is rotated, for example, for fifty four (54) degrees, each eighteen (18) degrees of the rotation both pawls 14.2 will simultaneously jump over corresponding two radial teeth of the outer toothed rim 15.3 of the driving part, wherein each of the radial teeth of the outer toothed rim 15.3 is functionally engaged with a corresponding one of the two pawls 14.2. When one of the two pawls 14.2 jumps over a corresponding one of the radial teeth of the outer toothed rim 15.3 of the driving part and another pawl 14.2 simultaneously jumps over another corresponding radial teeth of the radial teeth of the outer toothed rim 15.3 of the driving part, two separate sound signals or sounds in the form of clicks or cracks will be substantially simultaneously produced or generated, wherein the simultaneously generated sounds will overlap with each other so as to form a resulting sound signal or a sound having an increased volume which will be clearly or distinctively audible for the user and/or patient. Therefore, in case when the driving part 15 is rotated for fifty four (54) degrees, there will be created or generated three (3) separate resulting sound signals or a sound having an increased volume: a first resulting sound signal or a sound having an increased volume when rotating the driving part 15 for initial eighteen (18) degrees, a second resulting sound signal or a sound having an increased volume when rotating the driving part 15 for subsequent eighteen (18) degrees, i.e. when rotating the driving part 15 totally for thirty six (36) degrees, and a third resulting sound signal or a sound having an increased volume when rotating the driving part 15 for the final eighteen (18) degrees, i.e. when rotating the driving part 15 totally for fifty four (54) degrees, wherein both pawls 14.2 will finally perform three parallel or simultaneous jumps over corresponding radial teeth of the outer toothed rim 15.3 of the driving part.

In one of alternative embodiments of the present invention, the bearing sleeve 14 may be provided with two resilient elements, wherein each of the resilient elements may be similar to any of the resilient elements 14.1. Therefore, the bearing sleeve 14 may have two (2) pawls, wherein each of the pawls may be similar to any one of the pawls 14.2, and tips of the pawls may be circumferentially arranged and angularly shifted or offset by one hundred seventy four (174) degrees in relation to each other or relative to each other (i.e. an angle between the tips of the pawls 14.2 is one hundred seventy four degrees). The outer toothed rim 15.3 of the driving part may comprise thirty (30) inclined radial teeth which may be shifted or offset circumferentially of the toothed portion of the driving part 15 for twelve (12) degrees in relation to each other. In other words, each of the inclined radial teeth of the outer toothed rim 15.3 may be arranged each twelve (12) degrees on the external circumference of the toothed portion of the driving part 15. When the driving part 15 and the inner housing 11 having the driving part 15 partly inserted into the inner housing 11 are rotated together for six (6) degrees, only one of the two pawls 14.2 may substantially jump over a corresponding one of thirty (30) inclined radial teeth of the outer toothed rim 15.3 of the driving part. In other words, in the present alternative embodiment of the present invention, the pawls 14.2 may be circumferentially shifted or offset in relation to each other such that when rotating the driving part 15 for each six (6) degrees (a half of the angular pitch of the ratchet mechanism provided in the lower or distal portion of the syringe pen 100), said six degrees corresponding to a single unit of the drug dose, the engagement between only one of the two pawls 14.2 and a corresponding one of thirty (30) inclined radial teeth of the outer toothed rim 15.3 of the driving part results in the generation or production of a single sound in the form of a click or crack. Thus, each entire angular pitch of the ratchet mechanism provided in the lower or distal portion of the syringe pen 100, the entire angular pitch being twelve (12) degrees, there will be generated two separate sounds in the form of clicks or cracks, i.e. a single sound in the form of a click or crack when rotating the driving part 15 for initial six (6) degrees, and then one more sound in the form of a click or crack when rotating the driving part 15 for subsequent six (6) degrees, wherein said subsequent six (6) degrees of the rotation of the driving part 15 in combination with the initial six (6) degrees of the rotation of the driving part 15 form a single angular pitch of the ratchet mechanism. The ratchet mechanism is provided in the lower or distal portion of the syringe pen 100 and allows audible sounds in the form of clicks or other crack sounds to be produced or generated, thereby providing a sound feedback for the process of delivering the set drug dose to the dose injection site used for injecting the dose into the patient. In particular, to generate a single sound in the form of a click or crack as a result of engagement between only one of the two pawls 14.2 and a corresponding one of thirty (30) inclined radial teeth of the outer toothed rim 15.3 of the driving part when rotating the driving part 15 for each six (6) degrees, the pawls 14.2 are required to be shifted or offset circumferentially in relation to each other by an angle being equal to one hundred seventy four (174) degrees (i.e. 180 degrees minus 6 degrees). Therefore, in the present alternative embodiment of the present invention, there is provided an increased frequency of the generation of audible sounds in the form of clicks or other crack sounds when rotating the driving part 15 for six (6) degrees for delivering the single unit of the drug dose to the dose injection site.

In another alternative embodiment of the present invention, the bearing sleeve 14 may be provided with two resilient elements, wherein each of the resilient elements may be similar to any of the resilient elements 14.1. Therefore, the bearing sleeve 14 may have two (2) pawls, wherein each of the pawls may be similar to any one of the pawls 14.2, and tips of the pawls may be circumferentially arranged and angularly shifted or offset by one hundred seventy one (171) degrees in relation to each other or relative to each other (i.e. an angle between the tips of the pawls 14.2 is one hundred seventy one degrees). The outer toothed rim 15.3 of the driving part may comprise twenty (20) inclined radial teeth which may be shifted or offset circumferentially of the toothed portion of the driving part 15 for eighteen (18) degrees in relation to each other. In other words, each of the inclined radial teeth of the outer toothed rim 15.3 may be arranged each eighteen (18) degrees on the external circumference of the toothed portion of the driving part 15. When the driving part 15 and with the inner housing 11 having the driving part 15 partly inserted into the inner housing 11 are rotated together for nine (9) degrees, only one of the two pawls 14.2 may substantially jump over a corresponding one of twenty (20) inclined radial teeth of the outer toothed rim 15.3 of the driving part. In other words, in the present alternative embodiment of the present invention, the pawls 14.2 may be circumferentially shifted or offset in relation to each other such that when rotating the driving part 15 for each nine (9) degrees (a half of the angular pitch of the ratchet mechanism provided in the lower or distal portion of the syringe pen 100), said nine degrees corresponding to a single unit of the drug dose, the engagement between only one of the two pawls 14.2 and a corresponding one of twenty (20) inclined radial teeth of the outer toothed rim 15.3 of the driving part results in the generation or production of a single sound in the form of a click or crack. Thus, each entire angular pitch of the ratchet mechanism, provided in the lower or distal portion of the syringe pen 100, the entire angular pitch being eighteen (18) degrees, there will be generated two separate sounds in the form of clicks or cracks, i.e. a single sound in the form of a click or crack when rotating the driving part 15 for initial nine (9) degrees, and then one more sound in the form of a click or crack when rotating the driving part 15 for subsequent nine (9) degrees, wherein said subsequent nine (9) degrees of the rotation of the driving part 15 in combination with the initial nine (9) degrees of the rotation of the driving part 15 form a single angular pitch of the ratchet mechanism. The ratchet mechanism is provided in the lower or distal portion of the syringe pen 100 and allows audible sounds to be produced or generated in the form of clicks or other crack sounds, thereby providing a sound feedback for the process of delivering the set drug dose to the dose injection site used for injecting the dose into the patient. In particular, to generate a single sound in the form of a click or crack as a result of the engagement of only one of the two pawls 14.2 with a corresponding one of thirty (30) inclined radial teeth of the outer toothed rim 15.3 of the driving part when rotating the driving part 15 for each nine (9) degrees, the pawls 14.2 are required to be shifted or offset on the external circumference in relation to each other for an angle being equal to one hundred seventy one (171) degrees (i.e. 180 degrees minus 9 degrees). Therefore, in the present alternative embodiment of the present invention, there is provided an increased frequency of the generation of audible sounds in the form of clicks or other crack sounds when rotating the driving part 15 for nine (9) degrees for delivering the single unit of the drug dose to the dose injection site.

The sound feedback is generated by a tip of one of the pawls 14.2 passing over a top of a particular one of the radial teeth of the outer toothed rim 15.3 of the driving part as the inner housing 11 rotates together with the driving part 15 in the mode of delivering the set drug dose to the dose injection site used for injecting the dose into the patient. The generated sound feedback is generally represented by a set of sound signals in the form of clicks or crack sounds generated by the syringe pen 100 and allows the user, in particular a visually impaired user or a user with distractable attention, to control, by ear, the course of the process of delivering the set drug dose to the dose injection site used for injecting the dose into the patient, the dose injection site being substantially positioned on the patient body.

It is to note that the toothed rim 15.3 of the driving part having the inclined radial teeth and the resilient elements 14.1 of the bearing sleeve, the resilient elements 14.1 being each provided with a pawl 14.2 arranged to mesh with a corresponding one of the inclined radial teeth, form together a substantially sound-generating mechanism or a sound feedback mechanism indicating to the user, by means of audible sound signals in the form of clicks or cracks, the beginning, the continuation and/or the completion of the process of delivering the set drug dose to the dose injection site used for injecting the dose into the patient.

It is to further note that in the present invention at least the following aforementioned structural parts of the provided syringe pen 100 may be formed by a cylindrical part, a cylindrical member, a cylindrical housing, a tubular housing, a tube, a tubular member, a tubular part, a sleeve, a bush, or the like: the outer housing 1, the inner housing 11, the drug-dosing drum 5, the connecting tube 8, the inner housing 11, the bearing sleeve 14 and the driving part 15.

The above-described syringe pen 100 may be used for administering or injecting a drug into a mammal (for example, a human or an animal), the drug being selected from a group consisting of insulins, insulin analogs, hormones, heparins, antihistamines, and their derivatives and analogs.

## Claims

1. A drug delivery device, the device comprising:
an outer housing,
a drug reservoir installed in the outer housing,
an actuating mechanism designed to generate an actuating force,
a dose-setting mechanism installed at least partly in the outer housing, wherein the dose-setting mechanism is capable of setting a drug dose and functionally connected to the actuating mechanism,
an inner housing installed in the outer housing and functionally connected to the dose-setting mechanism, and
a rod movably installed at least partly in the inner housing so as to enable the drug to be discharged from the reservoir,
**characterized in that** the drug delivery device further comprises
a driving part provided with radial teeth, wherein the driving part is intermeshed in the outer housing with the inner housing and threadably engaged with rod such that the actuating force is transferred to the rod in order to move the latter depending on the set drug dose, and
a cylindrical part provided with at least one resilient element having a paw, wherein the cylindrical part is secured in the outer housing so as to enable the paw to jump over at least over one of the radial teeth of the driving part when rotating the driving part.

2. The device according to claim 1, wherein the resilient element is integral with the cylindrical part.

3. The device according to any of claims 1-2, wherein the cylindrical part is provided with two resilient elements each having a pawl, wherein the cylindrical part is secured such that the pawls are arranged around the driving part and angularly shifted by 180 degrees in relation to each other so as to enable both pawls to jump over corresponding radial teeth of the driving part when rotating the driving part.

4. The device according to claim 3, wherein each rotation of the driving part for a predetermined angle corresponding to a single unit of the drug dose is provided to enable both pawls to jump over corresponding radial teeth of the driving part.

5. The device according to claim 4, wherein the driving part comprises 20 radial teeth, and the predetermined rotation angle of the driving part is 18 degrees, and the radial teeth of the driving part are offset by 18 degrees, and the pawls of the resilient elements are offset by 180 degrees in relation to each other.

6. The device according to claim 1, wherein the pawls are circumferentially shifted in relation to each other such that each rotation of the driving part for a predetermined angle corresponding to a single unit of the drug dose is provided to enable one of the pawls to jump on a corresponding one of the radial teeth of the driving part.

7. The device according to claim 6, wherein the driving part comprises 30 radial teeth, and the predetermined rotation angle of the driving part is 6 degrees, and the angular shift between the pawls is 174 degrees.

8. The device according to claim 6, wherein the driving part comprises 20 radial teeth, and the predetermined rotation angle of the driving part is 9 degrees, and the angular shift between the pawls is 171 degrees.

9. Use of the drug delivery device according to any of claims 1-8 for injecting a drug into mammals, the drug being selected from a group consisting of insulins, insulin analogs, hormones, heparins, antihistamines, and their derivatives and analogs.
